# EUROPEAN PATENT SPECIFICATION

(11) **EP 4 149 583 B1**
(45) Date of publication and mention of the grant of the patent: **25.02.2026**
(21) Application number: 21725159.4
(22) Date of filing: 11.05.2021
(51) Int. Cl.: A61M 5/00

(54) **DRUG DELIVERY DEVICE**
ARZNEIMITTELABGABEVORRICHTUNG
DISPOSITIF D'ADMINISTRATION DE MÉDICAMENT

(30) Priority: 11.05.2020 DK PA202070302; 29.01.2021 DK PA202170040
(43) Date of publication of application: 22.03.2023
(73) Proprietor: Biograil APS, 4000 Roskilde (DK)
(72) Inventor: SKAK, Nikolaj, 4000 Roskilde (DK); LINDHARDT, Karsten, 4000 Roskilde (DK)
(74) Representative: Aera A/S
(86) International application number: PCT/EP2021/062436
(87) International publication number: WO 2021/228826

(56) References cited:
- WO-A1-2019/121686
- US-A1- 2006 293 625
- US-A1- 2015 051 589
- US-B2- 8 202 247

## Description

The present disclosure relates to a drug delivery device and in particular to a drug delivery device for oral administration. The drug delivery device is advantageously configured for delivery of an active drug substance in the gastrointestinal tract including the stomach and/or intestines, such as the small intestines and/or the large intestines (colon).

### BACKGROUND

A number of for example low permeable and/or low water soluble active drug substances are currently delivered by i.e. subcutaneous, intradermal, intramuscular, rectal, vaginal or intravenous route. Oral administration has the potential for the widest patient acceptance and thus attempts to deliver low permeable and/or low water soluble active drug substances through the preferred oral route of administration has been tried but with limited success in particular due to lack of stability and limited absorption from the gastrointestinal tract.

Stability both relates to the stability of the active drug substance during manufacturing and storage of the delivery device, and to the stability of the active drug substance during the passage in the gastrointestinal tract before it become available for absorption.

Limited gastrointestinal absorption is due to the gastrointestinal wall barrier preventing active drug substance from being absorbed after oral dosing because of the low permeability of the active drug substance, which is for example due to pre-systemic metabolism, size and/or the charges and/or because of the water solubility of the active drug substance.

Multiple approaches to solve these stability and absorption challenges have been suggested, but an effective solution to the challenges remain unresolved.

US 8,202.247 B2 relates to a capsule medical apparatus including a living tissue drawing portion in which a space for drawing the living tissue into a body of the capsule medical apparatus is formed; a movable unit that includes an engaging unit, which can be engaged with the living tissue of the subject, and that moves on a surface of the body of the capsule medical apparatus and in the living tissue drawing portion; an injection needle that has an ejection port for a drug and that protrudes such that the ejection port is positioned in the living tissue drawing portion; and an injection needle driver that drives the injection needle such that the injection needle protrudes.

US 2015/0051589 A1 relates to a capsule medical device and medical system, wherein the capsule type medical device is able to be suspended in a body cavity. The capsule type medical device performs continuous treatment while being suspended at a predetermined site.

### SUMMARY

Thus, there is an unmet need to provide a drug delivery device, which is capable of delivering drug substances for absorption in the gastrointestinal tissue. More generally, there remains a need for drug products and methods that enable enhanced drug delivery, when drug products are administered orally to patients.

A drug delivery device for oral drug delivery is disclosed, the drug delivery device having a central axis and comprising: a first body part; a first attachment part attached to the first body part, wherein the first attachment part has a first distal end provided with a tip configured to penetrate a biological tissue; and the drug delivery device comprising: a second attachment part having a second distal end; a second body part, wherein the second attachment part is attached to the second body part; and an actuator mechanism configured to move the first distal end towards the second distal end, and wherein the actuator mechanism is configured to rotate the first body part in relation to the second body part about a primary axis of the drug delivery device.

Also disclosed is a pharmaceutical composition comprising an active drug substance and one or more delivery devices as described herein.

It is an advantage of the present disclosure that the drug delivery device secures stability of the active drug substance during passage in the gastrointestinal tract and facilitates effective absorption of the active drug substance from the gastrointestinal tract after oral administration.

Further, it is advantage of the present disclosure that the drug delivery device provides an active attachment of the drug delivery device to the gastro-internal wall, such as the stomach wall and/or intestine wall.

Further, the present disclosure advantageously provides oral delivery of low permeable active drug substances in or at the gastro-internal tissue.

### BRIEF DESCRIPTION OF THE DRAWINGS

The above and other features and advantages of the present invention will become readily apparent to those skilled in the art by the following detailed description of exemplary embodiments thereof with reference to the attached drawings, in which:
Fig. 1 shows an exploded view of an exemplary drug delivery device,
Fig. 2 shows a sectional side view of an exemplary drug delivery device,
Fig. 3 shows a perspective view of an exemplary drug delivery device,
Fig. 4 shows a perspective view of an exemplary drug delivery device,
Fig. 5 shows a front view of an exemplary drug delivery device,
Fig. 6 shows a side view of an exemplary drug delivery device,
Fig. 7 shows a front view of an exemplary drug delivery device in engagement with a biological material,
Fig. 8 shows an encapsulated drug delivery device,
Fig. 9 shows an exploded view of an exemplary drug delivery device having rotatable attachment parts,
Fig. 10 shows a perspective view of an exemplary drug delivery device,
Figs. 11A-11D shows a schematic view of the operation of the drug delivery device,
Fig. 12 shows a drug delivery device in a first state,
Fig. 13 shows a drug delivery device in a second state,
Fig. 14 shows a drug delivery device in a first state,
Fig. 15 shows a drug delivery device in a second state,
Fig. 16 shows a drug delivery device,
Fig. 17 shows the drug delivery device of Fig. 16 in an exploded view,
Figs. 18-22 show experimental results using a drug delivery device, and
Fig. 23 shows pharmacodynamic data using a drug delivery device.

### DETAILED DESCRIPTION

Various exemplary embodiments and details are described hereinafter, with reference to the figures when relevant. It should be noted that the figures may or may not be drawn to scale and that elements of similar structures or functions are represented by like reference numerals throughout the figures. It should also be noted that the figures are only intended to facilitate the description of the embodiments and the functionalities associated therewith. They are not intended as an exhaustive description of the invention or as a limitation on the scope of the invention or the physical appearance of the invention. In addition, an illustrated embodiment needs not have all the aspects or advantages shown. An aspect or an advantage described in conjunction with a particular embodiment is not necessarily limited to that embodiment and can be practiced in any other embodiments even if not so illustrated, or if not so explicitly described.

A drug delivery device having a central axis is disclosed, the drug delivery device comprising a first body part; a first attachment part; a second attachment part; and an actuator mechanism configured to move the first attachment part and the second attachment part in relation to each other, e.g. by moving, such as rotating, the first body part in relation to a second body part.

The drug delivery device may have a size and geometry designed to fit into a pharmaceutical composition for oral administration.

The drug delivery device/pharmaceutical composition may be configured to be taken into the body via the oral orifice. Thus, the outer dimensions of the drug delivery device/pharmaceutical composition may be small enough for a user to swallow. The drug delivery device may be adapted to carry a drug substance into the body of the user, via the digestive system, so that the drug delivery device may e.g. travel from the mouth of the user into the stomach, via the oesophagus. The drug delivery device may further travel into the intestines from the stomach, and may optionally travel into the bowels and out through the rectum.

The drug delivery device may be configured to deliver the drug in any part of the digestive system of the user, where in one example it may be configured to deliver a drug substance into the stomach of the user. **In** another example, the drug delivery device may be adapted to initiate the drug delivery when the device has passed the stomach and has entered the intestine of the user. **In** other words, the drug delivery device may be configured to attach to a wall of the stomach or a wall of the intestines, e.g. depending on the desired release position of the active drug substance.

The attachment part(s) of the drug delivery device may be configured to interact with the inner surface linings of the gastrointestinal tract, so that the drug delivery device may e.g. be attached to the inner surface (mucous membrane) of the stomach, or alternatively to the mucous membrane of the intestines. The attachment part(s) may be configured to interact with the mucous membranes, e.g. in order to fix or attach the drug delivery device, e.g. for a period of time, inside the body of the user. By attaching the drug delivery device, it will allow a drug substance to be delivered into a part of the digestive system, in order to provide a drug substance to the body of the user. The attachment part(s) may be configured to interact with the mucous membranes, e.g. in order to inject drug substance into the gastrointestinal tract wall.

The drug delivery device has a central axis optionally extending from a first end to a second end of the drug delivery device. The drug delivery device may have a length (e.g. largest extension from first end to second end along central axis), in the range from 3 mm to 35 mm, such as in the range from 5 mm to 26 mm. The drug delivery device may be elongated.

The drug delivery device may have a width and/or height (e.g. largest extensions along width axis and height axis, respectively) in the range from 1 mm to 20 mm. Height and width are the largest extensions of the drug delivery device perpendicular to the central axis.

In one or more exemplary drug delivery devices, the dimensions of the drug delivery device, at least in an initial state or first state prior to actuation of the first attachment part and/or the second attachment part, may be represented by a length (largest extension along central axis), a width (largest extension along width axis perpendicular to the central axis) and a height (largest extension along height axis perpendicular to the central axis and the width axis). The height of the drug delivery device may be in the range from 1 mm to 15 mm. The width of the drug delivery device may be in the range from 1 mm to 15 mm.

In one or more exemplary drug delivery devices, the drug delivery device may be constructed in a way that secures the drug delivery part to deliver a payload or active drug substance into the internal tissue or internal surface for distribution of the active drug substance in the subject through the blood vessels.

Advantageously, the drug delivery device may be attached, and may deliver the active drug substance, to a particular location in a patient's intestinal wall. Of course, the delivery device may be attached, and may deliver the active drug substance, to other places as well. In one or more exemplary drug delivery devices, the drug delivery device, such as the spike, may penetrate the muscularis mucosa. In one or more exemplary drug delivery devices, the drug delivery device, such as the spike, may not penetrate the muscularis externa. In one or more exemplary drug delivery devices, the spike may be positioned in the submucosa. In one or more exemplary drug delivery devices, the spike may be positioned in the submucosa parallel to the gut wall.

The drug delivery device comprises a first body part. The first body part may be a two-part body part, i.e. the first body part may comprise a first primary body part and a first secondary body part. The first body part has an outer surface. A first primary recess and/or a first secondary recess may be formed in the outer surface of the first body part.

The drug delivery device optionally comprises a shell having a first shell part. An outer surface of the first body part may constitute at least a part of the first shell part.

The drug delivery device comprises a first attachment part. The first attachment part may comprise a first base part and/or a first needle, e.g. a spike. The first attachment part has a first proximal end and a first distal end. The first attachment part, such as the first needle or spike, optionally has or extends along a first attachment axis. A first tip of the first needle forms the first distal end. In other words, the first distal end is a first tip of the first needle. The first base may be arranged at or constitute the first proximal end of the first attachment part. The first needle may have a length in the range from 1 mm to 15 mm such, as in the range from 3 mm to 10 mm. Thereby sufficient penetration into the internal tissue may be provided for while at the same time reducing the risk of damaging the internal tissue. The first distal end of the first attachment part may be provided with a tip configured to penetrate a biological tissue. The first distal end of the first attachment part may be provided with a gripping part configured to grip a biological tissue.

The first needle may have a cross-sectional diameter in the range from 0.1mm to 5mm, such as in the range from 0.5mm to 2.0mm.

The first needle may be straight and/or curved. The first needle may comprise a first primary section that is straight. The first needle may comprise a first secondary section, e.g. between the first primary section and the first distal end or between the first base and the first primary section. The first secondary section may be curved.

The first needle may include two or more straight portions formed at an angle. For example, the first needle may have a proximal portion that extends at a first angle from a connection point to the drug delivery device and a distal portion that extends at a second angle from a connection point to the drug delivery device. The first angle and the second angle may be different. The proximal portion may connect to the distal portion at a joint (e.g., bend, connection, angle) and have a joint angle between the proximal portion and the distal portion. The joint angle may be an acute angle, an obtuse angle, or a right angle. The angle may be, for example, 10, 20, 30, 40, 50, 60, 70, 80, 90, 100, 120, 130 140, 150, 160, or 170 degrees. This can advantageously allow for different angles of attach when the first needle interacts with inner surface linings. This may allow for improved attachment of the drug delivery device, while helping to reduce or avoid tissue damage. Further, the joint may be flexible. Alternatively, the joint may not be flexible.

The joint may be located at a center, or generally at a center, of a length of the first needle. Alternatively, the joint may be located 40, 45, 55, 60, or 65% up a length of the first needle from the proximal end.

In one or more exemplary first attachment parts, the first needle may have three, four, or five different portions at different angles, each connected by a joint. **In** some iterations, any or all of the different portions may be straight or curved. Each joint may be flexible or not flexible.

The attachment parts of the drug delivery device may be seen as any kind of attachment parts that may be capable of attaching the drug delivery device to a biological tissue, such as a stomach wall, a wall of the bowels and/or intestines of a human or animal body. The attachment parts may be adapted to extend in a direction away from the central axis of the drug delivery device, and/or a central axis of the first attachment part. This may mean that the attachment part(s), e.g. at least in an activated state or second state of the drug delivery device, may extend in a direction away from a peripheral surface (in radial direction) of the first body part and/or the second body part, so that the attachment part extends farther in a radial direction than the peripheral or outer surface of the body part.

The first attachment part may be fixed or rotationally attached to the first body part.

In one or more exemplary drug delivery devices, the drug delivery device comprises a second body part. The second body part may be a two-part body part, i.e. the second body part may comprise a second primary body part and a second secondary body part. The second attachment part is optionally attached to the second body part. The second attachment part may be fixed or rotationally attached to the second body part. The second body part has an outer surface. A second primary recess and/or a second secondary recess may be formed in the outer surface of the second body part.

In one or more exemplary drug delivery devices, the actuator mechanism is configured to rotate the first body part in relation to the second body part about a primary axis of the drug delivery device. The primary axis may be parallel to and/or coinciding with the central axis.

In one or more exemplary drug delivery devices, the first body part is configured to rotate in a first direction and/or the second body part is configured to rotate in a second direction opposite to the first direction.

The drug delivery device may comprise a frame part, where different parts, such as the first body part and/or the second body part are attached, e.g. fixed or rotatably attached to the frame part. In one or more exemplary drug delivery devices, the actuator mechanism, or parts thereof, may be attached to the frame part. Thereby, separate rotation of the first body part and the second body part in relation to the frame part may be provided for

The rotational connection between the first body part and the second body part allows the first body part to rotate relative to the second body part, without the two parts separating from each other before the attachment part(s) interact with the internal tissue, such as mucous membranes. Such a connection may be obtained in a plurality of ways, where in one example the first body part has a plug connection and the second body part has a socket connection, where this plug and socket configuration allows the first body part to rotate relative to the second body part. A second example could be to provide an axle that may be coaxial with the central axis and/or the primary axis, where the first body part and the second body part are configured to receive the axle, and a stopping device is arranged at first and second ends of the axle, on each side of the combined first and second body part, preventing the first body part and the second body part to slide in a longitudinal direction along the axle. The axle may be integrated in the first body part or in the second body part.

If an axle were used, the axle can be made of any number of different materials. For example, the axle can be made of metals and/or alloys and/or polymers and/or composites and/or composites and/or combinations thereof.

The first and/or the second body part may be arranged to rotate freely relative to each other, e.g. at least in the second state, and thereby allowing the attachment parts to rotate relative to each other. Thus, the attachment parts may be adapted to come into contact and/or penetrate tissue of the gastrointestinal tract. The rotation of the body parts relative to each other using a resilient force may move the attachment parts in such a way that they are capable of e.g. penetrating or pinching the mucous membrane in order to fix the drug delivery device at a location in the gastrointestinal tract, such as the stomach or intestines. The penetrating and/or pinching force may come from the actuator mechanism/resilient part, where the resilient part may be adapted to store a resilient force that is capable of forcing the attachment parts towards each other when the resilient force of the resilient part has been at least partly unleashed. The resilient part may e.g. be in the form of a spring or spring element, for example a torsional spring or a power spring, where the spring may be wound up to store mechanical energy, where the mechanical energy may be transmitted to the first and/or the second body part. When the mechanical energy is released, the first body part may rotate relative to the second body part, and where the mechanical energy may be transferred into the attachment parts via the body parts.

Within the context of the present description the term "rotational force" may be seen as Torque, moment, moment of force, rotational force or "turning effect". Another definition of the term "rotational force" may be the product of the magnitude of the force and the perpendicular distance of the line of action of force from the axis of rotation. The rotational force may be seen as the force which is transferred from the resilient part to the attachment members of the drug delivery device via the body parts.

The rotational force may be defined as being large enough to penetrate into the gastrointestinal tissue. When the rotational force is applied to both the first and the second body part, the first attachment member may come into contact with the surface to be attached to, and where the rotational force applied to the second body part may cause the second attachment part to come into contact with the same surface, where the first attachment part provides a force, while the second attachment part provides a counter force to the first attachment part, so that the force is applied in such a manner that the first attachment part is forced in a direction towards the second attachment part, or vice versa.

In one or more exemplary drug delivery devices, a distance between the first attachment axis of the first attachment part and the primary axis, e.g. at least in an activated state or second state of the drug delivery device and optionally in an initial state of the drug delivery device, is larger than 0.5 mm.

In one or more exemplary drug delivery devices, a distance between the second attachment axis of the second attachment part and the primary axis, e.g. at least in an activated state or second state of the drug delivery device and optionally in an initial state of the drug delivery device, is larger than 0.5 mm.

In one or more exemplary drug delivery devices, the first attachment part is rotationally attached to the first body part, e.g. via a first joint connection having a first rotation axis. In other words, the first attachment part is optionally configured to rotate about a first rotation axis, e.g. in relation to the first body part. The first rotation axis may be parallel to the central axis and/or the primary axis. The first rotation axis may form a first angle with the central axis and/or the primary axis. The first angle may be less than 15°. The first angle may be in the range from 75° to 105°, such as 90°±5° or 90°.

In one or more exemplary drug delivery devices, the first body part may define a first body recess (e.g., cavity, slot, hole) extending to an outer surface of the first body part. The first body recess may be formed by solid walls on all sides except an outermost surface which is open. The first attachment part may be rotationally connected within the first body recess along a first attachment part axis. The first attachment part axis may be, for example, a pin (e.g., arm, support). The first attachment part axis may be parallel to the central axis and/or the primary axis. The first attachment part axis may be angled with respect to the central axis and/or the primary axis. Accordingly, the first attachment part may be configured to rotate within the recess along the first attachment part axis. Further, rotation of the first attachment part may be stopped at end surfaces of the recess.

The first body recess may extend along a portion of the outer surface of the first body. The first body recess may extend fully along an outer circumference of the first body. The first body recess may extend around 10, 15, 20, 25, 30, 35, 40, 45, 50, 55, 60, 65, 70, 75, 80, 85, 90, 95, or 100% of an outer circumference of the first body. The first body recess may extend around greater than 10, 15, 20, 25, 30, 35, 40, 45, 50, 55, 60, 65, 70, 75, 80, 85, 90, or 95% of an outer circumference of the first body. The first body recess may extend around less than 10, 15, 20, 25, 30, 35, 40, 45, 50, 55, 60, 65, 70, 75, 80, 85, 90, 95, or 100% of an outer circumference of the first body. The first body may optionally contain more than one first body recess, for example of a plurality of first attachment parts are used on the first body. If more than one first body recess is used, they may be spaced longitudinally apart and/or circumferentially apart.

The first body recess may extend from an outer surface toward the central axis through 5, 10, 15, 20, 25, 30, 35, or 40% of the drug delivery device. The first body recess may extend from an outer surface toward the central axis through greater than 5, 10, 15, 20, 25, 30, 35, or 40% of the drug delivery device. The first body recess may extend from an outer surface toward the central axis through less than 5, 10, 15, 20, 25, 30, 35, or 40% of the drug delivery device.

In one or more exemplary drug delivery devices, the first body recess may extend circumferentially, or partially circumferentially around the first body with the central axis being the longitudinal direction. The first body recess may extend perpendicularly with respect to the central axis and/or the primary access (e.g., may extend along a cross section of the drug delivery device perpendicular to the central axis and/or the primary access). The first body recess may have any number of shapes. For example, the first body recess can be a portion of a circle, such as a half circle. The first body recess can be a triangle. The first body recess can be a sector of a circle. The first body recess can be a curved edge connected by two straight edge. The first body recess can be two curved edges connected to each other by two straight edges.

Thus, the first attachment part may rotate on the first attachment part axis in order to move perpendicular to the central axis and/or the primary axis. In certain embodiments, the first attachment part may rotate at an angle between perpendicular and parallel with respect to the central axis and/or the primary axis.

In one or more exemplary drug delivery devices, when the first body part and/or the second body part rotate with respect to one another, the first attachment part and/or the second attachment part can rotate out of their respective recesses (e.g., first body recess and second body recess) due to the rotation of the first body part and/or the second body part. The continued rotation of the first body part and/or the second body part then causes the first attachment part and/or the second attachment part to pierce tissue to hold the drug delivery device in place.

In one or more exemplary drug delivery devices, the first attachment part extends, e.g. at least in an activated state of the drug delivery device and optionally in an initial state of the drug delivery device, in a direction away from the first body part. In other words, the first needle may extend, e.g. at least in an activated state of the drug delivery device and optionally in an initial state, from an outer surface of the first body part. Formulated differently, the first attachment axis may, e.g. at least in an activated state of the drug delivery device and optionally in an initial state of the drug delivery device form an angle of at least 45° with the central axis and/or the primary axis. An attachment part extending in a direction is to be understood as the direction from proximal end of attachment part/needle part to distal end of attachment part along the attachment axis of the attachment part.

The first attachment part may in a first state of the drug delivery device extend in a first primary direction and in a second state of the drug delivery device extend in a first secondary direction. The first primary direction and the first secondary direction may form an angle of at least 30°. The first primary direction may be parallel or substantially parallel to the central axis. The first primary direction may form an angle less than 60° with the central axis. The first secondary direction may form an angle of at least 60° such as about 90° with the central axis. The first secondary direction may be perpendicular to the central axis.

The first distal end of the first attachment part may be configured to move or be moved from a first primary position in a first state of the drug delivery device to a first secondary position in the second state.

The drug delivery device comprises a second attachment part. The second attachment part may comprise a second base part and/or a second needle, e.g. spike. The second attachment part has a second proximal end and a second distal end. The second attachment part, such as the second needle, optionally has or extends along a second attachment axis. A second tip of the second needle forms the second distal end. **In** other words, the second distal end is a second tip of the second needle. The second base may be arranged at or constitute the second proximal end of the second attachment part. The second needle may have a length in the range from 1 mm to 15 mm such, as in the range from 3 mm to 10 mm. Thereby sufficient penetration into the internal tissue may be provided for while at the same time reducing the risk of damaging the internal tissue. The second distal end of the second attachment part may be provided with a tip configured to penetrate a biological tissue. The second distal end of the second attachment part may be provided with a gripping part configured to grip a biological tissue.

The second needle may have a cross-sectional diameter in the range from 0.1mm to 5mm, such as in the range from 0.5mm to 2.0mm.

The second needle may be straight and/or curved. The second needle may comprise a second primary section that is straight. The second needle may comprise a second secondary section, e.g. between the second primary section and the second distal end or between the second base and the second primary section. The second secondary section may be curved.

The second needle may include two or more straight portions formed at an angle. For example, the second needle may have a proximal portion that extends at a first angle from a connection point to the drug delivery device and a distal portion that extends at a second angle from a connection point to the drug delivery device. The first angle and the second angle may be different. The proximal portion may connect to the distal portion at a joint (e.g., bend, connection, angle) and have a joint angle between the proximal portion and the distal portion. The joint angle may be an acute angle, an obtuse angle, or a right angle. The angle may be, for example, 10, 20, 30, 40, 50, 60, 70, 80, 90, 100, 120, 130 140, 150, 160, or 170 degrees. This can advantageously allow for different angles of attach when the second needle interacts with inner surface linings. This may allow for improved attachment of the drug delivery device, while helping to reduce or avoid tissue damage. Further, the joint may be flexible. Alternatively, the joint may not be flexible.

The joint may be located at a center, or generally at a center, of a length of the second needle. Alternatively, the joint may be located 40, 45, 55, 60, or 65% up a length of the second needle from the proximal end.

In one or more exemplary second attachment parts, the second needle may have three, four, or five different portions at different angles, each connected by a joint. In some iterations, any or all of the different portions may be straight or curved. Each joint may be flexible or not flexible.

In one or more exemplary drug delivery devices, both the first needle and the second needle include a joint. However, only one of the first needle and the second needle may include a joint with the other being straight and/or curved. If both the first needle and the second needle include a joint, the first distal tip and the second distal tip may be angled towards one another in order to facilitate attachment when the first body part and the second body part rotate with respect to one another.

In one or more exemplary drug delivery devices, the second attachment part is rotationally attached to the second body part, e.g. via a second joint connection having a second rotation axis. In other words, the second attachment part is optionally configured to rotate about a second rotation axis, e.g. in relation to the second body part. The second rotation axis may be parallel to the central axis and/or the primary axis. The second rotation axis may form a second angle with the central axis and/or the primary axis. The second angle may be less than 15°. The second angle may be in the range from 75° to 105°, such as 90°±5° or 90°.

In one or more exemplary drug delivery devices, the second body part may define a second body recess (e.g., cavity, slot, hole) extending to an outer surface of the second body part. The second body recess may be formed by solid walls on all sides except an outermost surface which is open. The second attachment part may be rotationally connected within the second body recess along a second attachment part axis. The second attachment part axis may be, for example, a pin (e.g., arm, support). The second attachment part axis may be parallel to the central axis and/or the primary axis. The second attachment part axis may be angled with respect to the central axis and/or the primary axis. Accordingly, the second attachment part can be configured to rotate within the recess along the second attachment part axis. Further, rotation of the second attachment part may be stopped at end surfaces of the second body recess.

The second body recess may extend along a portion of the outer surface of the second body. The second body recess may extend fully along an outer circumference of the second body. The second body recess may extend around 10, 15, 20, 25, 30, 35, 40, 45, 50, 55, 60, 65, 70, 75, 80, 85, 90, 95, or 100% of an outer circumference of the second body. The second body recess may extend around greater than 10, 15, 20, 25, 30, 35, 40, 45, 50, 55, 60, 65, 70, 75, 80, 85, 90, or 95% of an outer circumference of the second body. The second body recess may extend around less than 10, 15, 20, 25, 30, 35, 40, 45, 50, 55, 60, 65, 70, 75, 80, 85, 90, 95, or 100% of an outer circumference of the second body. The second body may optionally contain more than one second body recess, for example of a plurality of second attachment parts are used on the second body. If more than one second body recess is used, they may be spaced longitudinally apart and/or circumferentially apart.

The second body recess may extend from an outer surface toward the central axis through 5, 10, 15, 20, 25, 30, 35, or 40% of the drug delivery device. The second body recess may extend from an outer surface toward the central axis through greater than 5, 10, 15, 20, 25, 30, 35, or 40% of the drug delivery device. The second body recess may extend from an outer surface toward the central axis through less than 5, 10, 15, 20, 25, 30, 35, or 40% of the drug delivery device.

In one or more exemplary drug delivery devices, the second body recess may extend circumferentially, or partially circumferentially around the second body with the central axis being the longitudinal direction. The second body recess may extend perpendicularly with respect to the central axis and/or the primary access (e.g., may extend along a cross section of the drug delivery device perpendicular to the central axis and/or the primary access). The second body recess may have any number of shapes. For example, the second body recess can be a portion of a circle, such as a half circle. The second body recess can be a triangle. The second body recess can be a sector of a circle. The second body recess can be a curved edge connected by two straight edge. The second body recess can be two curved edges connected to each other by two straight edges.

Thus, the second attachment part may rotate on the second attachment part axis in order to move perpendicular to the central axis and/or the primary axis. In certain embodiments, the second attachment part may rotate at an angle between perpendicular and parallel with respect to the central axis and/or the primary axis.

In one or more exemplary drug delivery devices, when the first body part and/or the second body part rotate with respect to one another, the first attachment part and/or the second attachment part can rotate out of their respective recesses (e.g., first body recess and second body recess) due to the rotation of the first body part and/or the second body part. The continued rotation of the first body part and/or the second body part then causes the first attachment part and/or the second attachment part to pierce tissue to hold the drug delivery device in place.

In one or more exemplary drug delivery devices, the second attachment part extends, e.g. at least in an activated state of the drug delivery device and optionally in an initial state of the drug delivery device, in a direction optionally away from the second body part. In other words, the second needle may extend, e.g. at least in an activated state of the drug delivery device and optionally in an initial state, from an outer surface of the second body part. Formulated differently, the second attachment axis may, e.g. at least in an activated state of the drug delivery device and optionally in an initial state of the drug delivery device form an angle of at least 45° with the central axis and/or the primary axis.

The second attachment part may in a first state of the drug delivery device extend in a second primary direction and in a second state of the drug delivery device extend in a second secondary direction. The second primary direction and the second secondary direction may form an angle of at least 30°. The second primary direction may be parallel or substantially parallel to the central axis. The second primary direction may form an angle less than 60° with the central axis. The second secondary direction may form an angle of at least 60°, such as about 90° with the central axis. The second secondary direction may be perpendicular to the central axis.

The second distal end of the second attachment part may be configured to move or be moved from a second primary position in a first state of the drug delivery device to a second secondary position in the second state.

The drug delivery device comprises an actuator mechanism. The actuator mechanism is configured to move the first attachment part in relation to the second attachment part, such as configured to move the first distal end towards and/or away from the second distal end, e.g. at least during a part of a rotation, such as in a first rotation and optionally in a second rotation. To move the first distal end towards the second distal end may be understood as reducing a distance between the first distal end and the second distal end. To move the first distal end towards the second distal end may be understood as reducing an angle between the first attachment axis and the second attachment axis, such as reducing an angle between a first secondary direction of the first attachment part and a second secondary direction of the second attachment part. In one or more exemplary drug delivery devices, the actuator mechanism is configured to move the first distal end towards the second distal end by rotating, e.g. in a second state of the drug delivery device, the first body part in relation to the second body part and/or vice versa. The actuator mechanism may be configured to rotate the first body part at least 90°, such as at least 450°, at least 810°, at least 1170°, at least 1530°, or even at least 1890° in relation to the second body part about the primary axis. The actuator mechanism may be configured to rotate the first body part in relation to the second body part about the primary axis in a stepwise manner. In other words, to rotate the first body part in relation to the second body part about the primary axis may comprise a plurality of rotations including a first rotation and a second rotation, e.g. a first rotation followed by a first time period with reduced or no rotation followed by a second rotation. A first rotation followed by a second rotation after a first time period may increase the possibility of the drug delivery device attaching to the biological tissue. The first time period or in general time periods between rotations allows the drug delivery to move to other positions in the gastrointestinal tract. In other words, if the drug delivery does not attach to the biological tissue during a first rotation, further rotations increases the chance of attachment to the internal tissue. The first rotation may be at least 90°, and the second rotation may be at least 180°. The plurality of rotations may comprise a third rotation. The third rotation may be at least 180°.

In one or more exemplary drug delivery devices, a movement of the first distal end towards the second distal end may be preceded by and/or followed by a movement of the first distal end away from the second distal end. In other words, a movement of the first distal end towards the second distal end may be prior to and/or after movement of the first distal end away from the second distal end. For example, a first rotation may comprise moving the first distal end towards the second distal end and/or moving the first distal end away from the second distal end. For example, a second rotation may comprise moving the first distal end towards the second distal end and/or moving the first distal end away from the second distal end. For example, a third rotation may comprise moving the first distal end towards the second distal end and/or moving the first distal end away from the second distal end.

The actuator mechanism optionally comprises a resilient part such as a spring element configured to apply force to the first body part and/or the second body part. The resilient part may comprise a first part, such as a first end, connected to the first body part. The resilient part may comprise a second part, such as a second end, connected to the second body part.

**In** one or more exemplary drug delivery devices, the actuator mechanism optionally comprises a swelling media, i.e. a media increasing its volume, e.g. upon contact with a fluid, e.g. in order to provide rotation of parts in relation to each other. **In** one or more exemplary drug delivery devices, a swelling medial provides rotation of the first attachment part in relation to the first body part and/or provides rotation of the second attachment part in relation to the second body part. **In** one or more exemplary drug delivery devices, a swelling medial provides rotation of the first body part in relation to the second body part.

The actuator mechanism, such as the resilient part, may be configured to rotate the first attachment part about the first rotation axis in relation to the first body part.

The actuator mechanism, such as the resilient part, may be configured to rotate the second attachment part about the second rotation axis in relation to the second body part.

**In** one or more exemplary drug delivery devices, the drug delivery device comprises a first compartment, the drug delivery device being configured to deliver an active drug substance from the first compartment to the surroundings of the drug delivery device. The first compartment may be arranged in the first attachment part, such as in the first needle, e.g. within a distance of 8 mm, such as within 5 mm, from the first distal end. The first attachment part, such as the first needle may have one or more openings providing access to the first compartment. **In** one or more exemplary drug delivery devices, the first compartment is formed as a through-going bore in the first needle.

The first compartment may be arranged in any part of the drug delivery device, such as in the form of a cavity inside a volume of the first body part, the second body part or both of the first body part and the second body part. Additionally, or alternatively, the first compartment may be a compartment which is inside the first attachment part, where the penetration of the first attachment part into a biological tissue may release a drug substance in the first compartment into the biological tissue. Additionally or alternatively, the first compartment may be compartment that is in the form of a depression or opening or spike or hollow spike on the outer surface of the first and/or the second body part, where the drug delivery device may be adapted to release the drug substance inside the organ of the body which the drug delivery device is adapted to pass through.

In one or more exemplary drug delivery devices, the first compartment may be open from an inner volume of the drug delivery device and towards an outer part of the drug delivery device. In one or more examples, the first compartment may be inside the first body part, and where the first compartment is in fluid connection with the first attachment part, so that when the first distal end of the first attachment part has penetrated the biological tissue, the drug substance may be released from the first compartment and into the biological tissue via the first attachment part. This may e.g. be where the first attachment part is a tubular part, which has a first distal end in fluid communication with the first compartment of the drug delivery device.

In one or more exemplary drug delivery devices, the drug delivery device comprises a second compartment, the drug delivery device being configured to deliver an active drug substance from the second compartment to the surroundings of the drug delivery device. The second compartment may be arranged in the first attachment part or in the second attachment part, such as in the second needle, e.g. within a distance of 8 mm, such as within 5 mm, from the second distal end. The second attachment part, such as the second needle may have one or more openings providing access to the second compartment. In one or more exemplary drug delivery devices, the second compartment is formed as a through-going bore in the first needle or in the second needle.

In one or more exemplary drug delivery devices, the first attachment part and the second attachment part form an angle when the first distal end and the second distal end are in a plane that includes the primary axis. In other words, the first attachment axis and the second attachment may form an angle, e.g. larger than 5°, such as in the range from 10° to 75°, when the first distal end and the second distal end are in a plane that includes the primary axis.

In one or more exemplary drug delivery devices, the drug delivery device has a first state, also denoted initial state, where the first body part and the second body part are rotationally stationary relative to each other and a second state, also denoted activated state, where the first body part and the second body part are rotationally mobile relative to each other, e.g. can rotate about the primary axis of the drug delivery device. In other words, the first body part may be locked, e.g. prevented from rotating, in relation to the second body part. The first state may e.g. be an initial state or introduction state, where the drug delivery device is adapted to be introduced into the body, and where the first body part and the second body part are stationary relative to each other. In the first state the resilient part may have a predefined amount of stored energy, where the energy level is stationary in the resilient part while the body parts are stationary.

In one or more exemplary drug delivery devices, the drug delivery device has a first state where the resilient part has a constant resilient force load and a second state where the resilient part at least partly releases the resilient force load. **In** other words, the resilient part may be biased or preloaded in the first state of the drug delivery and upon release, e.g. by release of a locking mechanism, (i.e. the drug delivery device being in the second state) the force from the resilient part may effect a rotation of the first body part in relation to the second body part, i.e. including a movement of the first distal end towards the second distal.

In one or more exemplary drug delivery devices, the actuator mechanism is configured to move the first distal end from a first primary position, e.g. in first state of the drug delivery device, with a first primary radial distance from a central axis of the delivery device to a first secondary position, e.g. in second state of the drug delivery device, with a first secondary radial distance from the central axis and/or primary axis, wherein the first secondary radial distance is larger than the first primary radial distance. Thus, the first distal end of the first attachment may be in a first primary position when the drug delivery device is in the first state and/or the first distal end of the first attachment part may be in a first secondary position when the drug delivery device is in the second state.

The first primary radial distance may be less than 10 mm, such as less than 8 mm or even less than 5 mm. The first secondary radial distance may be larger than the first primary radial distance. The first secondary radial distance may be larger than 5 mm, such as larger than 6 mm, or larger than 8 mm. **In** one or more exemplary drug delivery devices, the first secondary radial distance is in the range from 6 mm to 15 mm.

In one or more exemplary drug delivery devices, the first attachment part, such as a part of the first needle and/or the first distal end, may, in the first state be arranged or at least partly arranged within a first primary recess of the first body part. **In** the first state, the first distal end may be arranged inside the first body part.

In one or more exemplary drug delivery devices, the first attachment part, such as a part of the first needle and/or the first distal end, may, in the second state be arranged or at least partly arranged outside the first primary recess of the first body part.

In one or more exemplary drug delivery devices, the first attachment part, such as a part of the first needle and/or the first distal end, may, in the first state be arranged within a second primary recess of the second body part. Thereby, the first attachment part may be configured to lock the first body part in relation to the second body part in the first state of the drug delivery device.

In one or more exemplary drug delivery devices, the first attachment part, such as a part of the first needle and/or the first distal end, may, in the second state be arranged outside the second body part and/or at least outside the second primary recess of the second body part.

In one or more exemplary drug delivery devices, the actuator mechanism is configured to move the second distal end from a second primary position, e.g. in first state of the drug delivery device, with a second primary radial distance from a central axis of the delivery device to a second secondary position, e.g. in second state of the drug delivery device, with a second secondary radial distance from the central axis and/or primary axis, wherein the second secondary radial distance is larger than the second primary radial distance. Thus, the second distal end of the second attachment may be in a second primary position when the drug delivery device is in the first state and/or the second distal end of the second attachment part may be in a second secondary position when the drug delivery device is in the second state.

The second primary radial distance may be less than 10 mm, such as less than 8 mm or even less than 5 mm. The second secondary radial distance may be larger than the second primary radial distance. The second secondary radial distance may be larger than 5 mm, such as larger than 6 mm, or larger than 8 mm. **In** one or more exemplary drug delivery devices, the second secondary radial distance is in the range from 6 mm to 15 mm.

In one or more exemplary drug delivery devices, the second attachment part, such as a part of the second needle and/or the second distal end, may, in the first state be arranged or at least partly arranged within a first secondary recess of the first body part. Thereby, the second attachment part may be configured to lock the first body part in relation to the second body part in the first state of the drug delivery device. In the first state, the second distal end may be arranged inside the second body part.

In one or more exemplary drug delivery devices, the second attachment part, such as a part of the second needle and/or the second distal end, may, in the second state be arranged or at least partly arranged outside the first body part and/or at least outside the first secondary recess of the first body part.

In one or more exemplary drug delivery devices, the second attachment part, such as a part of the second needle and/or the second distal end, may, in the first state be arranged within a second secondary recess of the second body part.

In one or more exemplary drug delivery devices, the second attachment part, such as a part of the second needle and/or the second distal end, may, in the second state be arranged outside the second secondary recess of the second body part.

In one or more exemplary drug delivery devices, the actuator mechanism is configured to move, e.g. by rotation about a first rotation axis of the first attachment part (first base part), the first distal end from a first primary angular position of first primary position to a first secondary angular position of first secondary position in relation to a first proximal end of the first attachment part. An angle between the first primary angular position and the first secondary angular position may be larger than 10°, such as larger than 45° or larger than 60°.

In one or more exemplary drug delivery devices, the actuator mechanism is configured to move, e.g. by rotation about a second rotation axis of the second attachment part (second base part), the second distal end from a second primary angular position of second primary position to a second secondary angular position of second secondary position in relation to a second proximal end of the second attachment part. An angle between the second primary angular position and the second secondary angular position may be larger than 10°, such as larger than 45° or larger than 60°.

In one or more exemplary drug delivery devices, the drug delivery device comprises a locking mechanism. The locking mechanism may be configured to lock, e.g. prevent rotation of, the first body part in relation to the second body part in a first state of the drug delivery device. The locking mechanism may be configured to lock the first attachment part in a first primary position, e.g. in relation to the first body part, when the drug delivery device is in the first state. Upon release of the locking mechanism, the first attachment part may be allowed to move from a first primary position to a first secondary position. The locking mechanism may upon release be configured to allow rotation of the first body part in relation to the second body part, e.g. in a second state of the drug delivery device. The locking mechanism may be configured to lock the second attachment part in a second primary position, e.g. in relation to the second body part, when the drug delivery device is in the first state. The locking mechanism may upon release be configured to allow the second attachment part to move from a second primary position to a second secondary position.

The locking mechanism may comprise a first locking element optionally configured to lock and/or unlock (release) the first body part in relation to the second body part. The first locking element may be configured to lock and/or unlock (release) the first attachment part in relation to the first body part. The first locking element may be configured to lock and/or unlock (release) the second attachment part in relation to the second body part. The first locking element may be arranged in a first primary recess of the first body part and/or in a second primary recess of the second body part. The first locking element may be configured to dissolve when the drug delivery device enters the gastrointestinal tract or at a desired location in the gastrointestinal tract, thereby releasing the first body part in relation to the second body part and allowing the actuator mechanism to rotate the first body part in relation to the second body part and thereby moving the first distal end towards the second distal end in turn resulting in attachment of the drug delivery device to the internal tissue.

The first locking element may be a first locking band (e.g., ring, loop, partial ring, partial loop). The first locking band may have a circumferential length greater than a longitudinal width. For example, the circumferential length may be 2x, 3x, 4x, 5x, 6x, 7x, 8x, 9x, or 10x the longitudinal width.

The first locking band may fit on an outer surface of the drug delivery device. For example, the first locking band may be located on an outer surface of the first body part or the second body part. The first locking band may be mechanically fit onto the drug delivery device. For example, the first locking band may be snap fit onto the drug delivery device. The first locking band may be chemically attached onto the drug delivery device.

In one or more exemplary drug delivery devices, the first locking band could be in the shape of a capsule part. For example, the first locking band could form a first half of a capsule. The first locking band could form a first half of a capsule and a second locking band could form a second half of the capsule. When fitted together, the first locking band and the second locking band could form a full capsule.

The first locking band may partially or fully cover the first body recess if located on the first body. Thus, the first locking band may prevent motion of the first attachment part. The first locking band may partially or fully cover the second body recess if located on the second body. Thus, the first locking band may prevent motion of the second attachment part. The first locking band may partially or fully cover both the first body recess and the second body recess. The first locking band may partially or fully cover the first body recess and a second locking band may partially or fully cover the second body recess.

The first locking band may extend fully along an outer circumference of the drug delivery device. The first locking band may extend around 10, 15, 20, 25, 30, 35, 40, 45, 50, 55, 60, 65, 70, 75, 80, 85, 90, 95, or 100% of an outer circumference of the drug delivery device. The first locking band may extend around greater than 10, 15, 20, 25, 30, 35, 40, 45, 50, 55, 60, 65, 70, 75, 80, 85, 90, or 95% of an outer circumference of the drug delivery device. The first locking band may extend around less than 10, 15, 20, 25, 30, 35, 40, 45, 50, 55, 60, 65, 70, 75, 80, 85, 90, 95, or 100% of an outer circumference of the drug delivery device.

In one or more exemplary drug delivery devices, the first locking band may include one or more locking protrusions (e.g., extensions, tabs, fingers, projections, teeth). For example, the first locking band may include 1, 2, 3, 4, 5, 6, 7, 8, 9, or 10 locking protrusions. The locking protrusions may only extend longitudinally from one side of the first locking band. The locking protrusions may extend longitudinally from both sides of the first locking band. The locking protrusions may be equally spaced along the first locking band. The locking protrusions may be unequally spaced along the first locking band.

The one or more locking protrusions may extend towards a longitudinal center of the drug delivery device (e.g., along an outer surface of the body towards the second body part if the first locking band is located on the first body part, or along an outer surface of the body towards the first body part if the first locking band is located on the second body part).

The one or more locking protrusions may be triangular, square, rectangular, rounded, or other polygonal shapes. The one or more locking protrusions may vary in shape along the first locking band.

**In** one or more exemplary drug delivery devices, the drug delivery device may include mating features. The mating features can be configured to mate with the one or more protrusions of the first locking band. The mating features can include one or more mating protrusions (e.g., extensions, tabs, fingers, projections, teeth) extending radially outward from an outer surface of the drug delivery device. The mating features can extend from the first body part, the second body part, or both. The mating features can be formed in one or more circumferential rows. For example, there can be one circumferential row of mating features or two circumferential rows of mating features. Both circumferential rows can be on the same body part (e.g., the first body part or the second body part). In alternative implementations, one circumferential row of mating features can be on the first body part and a second circumferential row of mating features can be on the second body part.

The one or more mating protrusions may be triangular, square, rectangular, rounded, or other polygonal shapes. The one or more mating protrusions may vary in shape along the first locking band. The one or more mating protrusions may be angled in a circumferential direction to form a mating recess (e.g., curve, cavity, space, gap). This mating recess can help lock the one or more mating protrusions to the one or more protrusions of the first locking band. Further, the mating recess can prevent unwanted release of the first locking band. Accordingly, when the first locking band is attached to the drug delivery device, the one or more locking protrusions can fit between the one or more mating protrusions. The one or more locking protrusions can fit within adjacent mating protrusions. This can prevent rotation of the first body with respect to the second body. For example, the first body will be impeded in rotating by the first locking band. **In** some embodiments, the locking protrusion can be located between two mating protrusions, each of the mating protrusions angled in opposite directions to hold the locking protrusion in place.

In some implementations the mating features may be recesses extending internally into the drug delivery device. The locking protrusions can then extend radially inward instead of longitudinally to mate with the mating features.

As discussed above, when the first locking band is attached and the one or more protrusions mate with the mating features, the first body part is locked in place with relation to the second body part. The first body part and the second body part may be released from the first locking band when the first locking band dissolves as discussed herein.

Further, the dissolving of the first locking band can allow the first attachment part or second attachment part to further rotate out of one of the first body recess or second body recess. Thus, when the first body and the second body rotate with respect to one another, the first attachment part and the second attachment part can rotate for inserting into tissue.

In one or more exemplary drug delivery devices, the first locking band can include one or a plurality of square-shaped locking protrusions and/or one or a plurality of triangle-shaped locking protrusions. The square-shaped locking protrusions may be used to keep the cover in place under the force of the mating protrusions. The triangle-shaped locking protrusions may be used to properly locate the first locking band.

In one or more exemplary drug delivery systems, the whole of
the first locking band can be dissolvable. In one or more exemplary drug delivery systems, only the square-shaped locking protrusions may be formed from a dissolvable material. Once the square-shaped locking protrusions dissolve, the first body part and the second body part may be allowed to rotate. Rotation of the first body part with respect to the second body part can cause the first locking band to translate, e.g. move, change position, relocate. This can occur as the mating protrusions can squeeze the triangle-shaped locking protrusions, pushing them longitudinally away. For example, rotation can cause the first locking band to translate along the central axis.

This translation can expose the first attachment part and/or the second attachment part, depending on the coverage of the first locking band. The translation can fully translate the first locking band off of the drug delivery device. The translation can partially translate the first locking band to expose the first attachment part and/or the second attachment part with the first locking band remaining associated with, e.g. attached to, the drug delivery device.

The locking mechanism may comprise a second locking element optionally configured to lock and/or unlock (release) the first body part in relation to the second body part. The second locking element may be configured to lock and/or unlock (release) the second attachment part in relation to the second body part. The second locking element may be arranged in a first secondary recess of the first body part and/or in a second secondary recess of the second body part. The second locking element may be configured to dissolve when the drug delivery device enters the gastrointestinal tract, thereby unlocking or releasing the first body part in relation to the second body part and allowing the actuator mechanism to rotate the first body part in relation to the second body part and thereby moving the first distal end towards the second distal end in turn resulting in attachment of the drug delivery device to the internal tissue.

One or more exemplary drug delivery devices may include a first cover band (e.g., ring, loop, partial ring, partial loop). The first cover band can be used in conjunction with the first locking element, e.g. locking element, locking mechanism. In one or more exemplary drug delivery devices, the first cover band can be the first locking band. In one or more exemplary drug delivery devices, the first cover band can include any or all features discussed above with respect to the first locking band. The first cover band may have a circumferential length greater than a longitudinal width. For example, the circumferential length may be 2x, 3x, 4x, 5x, 6x, 7x, 8x, 9x, or 10x the longitudinal width.

The first cover band may fit on an outer surface of the drug delivery device. For example, the first cover band may be located on an outer surface of the first body part or the second body part.

In one or more exemplary drug delivery devices, the first cover band could be in the shape of a capsule part. For example, the first cover band could form a first half of a capsule. The first cover band could form a first half of a capsule and a second cover band could form a second half of the capsule. When fitted together, the first cover band and the second cover band could form a full capsule.

The first cover band may be mechanically fit onto the drug delivery device. For example, the first cover band may be snap fit onto the drug delivery device. The first cover band may be chemically attached onto the drug delivery device.

The first cover band may partially or fully cover the first body recess if located on the first body. Thus, the first cover band may prevent motion of the first attachment part. The first cover band may partially or fully cover the second body recess if located on the second body. Thus, the first cover band may prevent motion of the second attachment part. The first cover band may partially or fully cover both the first body recess and the second body recess. The first cover band may partially or fully cover the first body recess and a second cover band may partially or fully cover the second body recess.

The first cover band may extend fully along an outer circumference of the drug delivery device. The first cover band may extend around 10, 15, 20, 25, 30, 35, 40, 45, 50, 55, 60, 65, 70, 75, 80, 85, 90, 95, or 100% of an outer circumference of the drug delivery device. The first cover band may extend around greater than 10, 15, 20, 25, 30, 35, 40, 45, 50, 55, 60, 65, 70, 75, 80, 85, 90, or 95% of an outer circumference of the drug delivery device. The first cover band may extend around less than 10, 15, 20, 25, 30, 35, 40, 45, 50, 55, 60, 65, 70, 75, 80, 85, 90, 95, or 100% of an outer circumference of the drug delivery device.

In one or more exemplary drug delivery devices, the first cover band may include one or more mating protrusions (e.g., extensions, tabs, fingers, projections, teeth). For example, the first cover band may include 1, 2, 3, 4, 5, 6, 7, 8, 9, or 10 mating protrusions. The mating protrusions may only extend longitudinally from one side of the first cover band. The mating protrusions may extend longitudinally from both sides of the first cover band. The mating protrusions may be equally spaced along the first cover band. The mating protrusions may be unequally spaced along the first cover band.

The one or more mating protrusions may extend towards a longitudinal center of the drug delivery device (e.g., along an outer surface of the body towards the second body part if the first cover band is located on the first body part, or along an outer surface of the body towards the first body part if the first cover band is located on the second body part).

In one or more exemplary drug delivery devices, the drug delivery device may include mating features. The mating features can be configured to mate, e.g. receive, hold, contact, with the one or more mating protrusions of the first cover band. The mating features can include one or more body mating protrusions (e.g., extensions, tabs, fingers, projections, teeth) extending radially outward from an outer surface of the drug delivery device. The mating features can extend from the first body part, the second body part, or both. The mating features can be formed in one or more circumferential rows. For example, there can be one circumferential row of mating features or two circumferential rows of mating features. Both circumferential rows can be on the same body part (e.g., the first body part or the second body part). In alternative implementations, one circumferential row of mating features can be on the first body part and a second circumferential row of mating features can be on the second body part.

The one or more mating protrusions may be triangular, square, rectangular, rounded, or other polygonal shapes. The one or more mating protrusions may vary in shape along the first cover band. The one or more mating protrusions may be angled in a circumferential direction to form a mating recess (e.g., curve, cavity, space, gap). This mating recess can help fit the one or more body mating protrusions to the one or more protrusions of the first cover band. Further, the mating recess can prevent unwanted release of the first cover band.

Accordingly, when the first cover band is attached to the drug delivery device, the one or more mating protrusions can fit between the one or more mating features. The one or more mating protrusions can fit within adjacent mating features. This can help properly align the first cover band.

In some implementations the mating features may be recesses extending internally into the drug delivery device. The mating protrusions can then extend radially inward instead of longitudinally to mate with the mating features. In one or more exemplary drug delivery devices, rotation of the first body part with respect to the second body part can cause the cover band to translate, e.g. move, change position, relocate. For example, rotation can cause the cover band to translate along the central axis. This translation can expose the first attachment part and/or the second attachment part, depending on the coverage of the cover band. This can occur, for example, as the mating protrusions can squeeze triangle-shaped mating protrusions, or other shaped mating protrusions, pushing them longitudinally away. The translation can fully translate the cover band off of the drug delivery device. The translation can partially translate the cover band to expose the first attachment part and/or the second attachment part with the cover band associated with, e.g. attached to, the drug delivery device.

In one or more exemplary drug delivery devices, the first cover band may be dissolvable. The dissolving of the first cover band can allow the first attachment part or second attachment part to further rotate out of one of the first body recess or second body recess. Thus, when the first body and the second body rotate with respect to one another, the first attachment part and the second attachment part can rotate for inserting into tissue. The material and/or properties of the first locking element and/or the second locking element may be selected such that the release of the body parts and/or activation of the drug delivery is controlled to take place at a desired location in the gastrointestinal tract, such as in the stomach or in the intestines. The material of the first locking element and/or the second locking element may comprise one or more of sugars, sugar derivatives, hydrophilic polymers, pH dependent polymers, and pharmaceutically acceptable excipients that disperses, dissolves, swells, and/or gels upon contact with water/fluid.

In one or more exemplary drug delivery devices, at least part of the first attachment part and/or the second attachment part may be made of a biodegradable material, absorbable material, or similar material which allows the material of the attachment part to be broken down, degraded and/or dissolved by processes that are present in the body, such as corrosion, degradation, hydrolysis and/or proteolytic enzymatic degradation. Thus, when the attachment part(s) has been inside the human body for a period of time, the attachment part(s) may dissolve, decompose or degrade to such a degree that the attachment part may lose its structural stability, which may in turn release the drug delivery device from the surface it has attached itself to. Thus, after a period, e.g. when the drug substance has been released from the attachment part(s), the attachment part(s) may deteriorate to such a degree that the drug delivery device may be released and may continue its journey through the gastrointestinal tract to be released through natural intestinal and/or bowel movements of the user or patient.

In one or more exemplary drug delivery devices, the rotational axis of the first body part and/or the second body part (primary axis) may be the central axis of the drug delivery device, e.g. the primary axis of the first body part may be coaxial to the central axis. Thus, the central axis intersects both the first body part and the second body part, and may define the primary axis.

In one or more exemplary drug delivery devices, the first body part and the second body part may be substantially symmetrical in a radial direction perpendicular to the central axis. This may mean that the first body part and/or the second body part may have a circular periphery, where the periphery may extend in a radial direction away from and perpendicular to the central axis.

The first attachment axis may be seen as an axis that is coaxial with the length of the first attachment part. The second attachment axis may be seen as an axis that is coaxial with the length of the second attachment part. In case the first attachment part has a shape that is not straight, the first attachment axis may be defined as an axis that intersects the first distal end and the first proximal end of the first attachment part. In case the second attachment part has a shape that is not straight, the second attachment axis may be defined as an axis that intersects the second distal end and the second proximal end of the second attachment part.

In one or more exemplary drug delivery devices, the first attachment axis may be positioned at a first distance from the central axis, while the second attachment axis may be positioned at a second distance from the central axis and/or primary axis.

For example, the first attachment axis may be positioned at a first primary distance from the central axis in the first state of the drug delivery device. The first primary distance may be larger than 0.5 mm, such as in the range from 1 mm to 15 mm or larger than 1 mm, e.g. 2 mm, 3 mm, 4 mm, 5 mm, 6mm, 7 mm, 8mm, 9mm, 10mm, 11mm, 12mm, 13 mm, or 14 mm.

The first attachment axis may cross or be close to (distance less than 0.5 mm) the central axis in the first state of the drug delivery device.

The first attachment axis may be positioned at a first secondary distance from the central axis in the second state of the drug delivery device. The first secondary distance may be larger than 0.5 mm, such as in the range from 1 mm to 15 mm or larger than 1 mm, e.g. 2 mm, 3 mm, 4 mm, 5 mm, 6mm, 7 mm, 8mm, 9mm, 10mm, 11mm, 12mm, 13 mm, or 14 mm.

The first attachment axis may cross or be close to (distance less than 0.5 mm) the central axis in the second state of the drug delivery device.

For example, the second attachment axis may be positioned at a second primary distance from the central axis in the first state of the drug delivery device. The second primary distance may be larger than 0.5 mm, such as in the range from 1 mm to 15 mm or larger than 1 mm, e.g. 2 mm, 3 mm, 4 mm, 5 mm, 6mm, 7 mm, 8mm, 9mm, 10mm, 11mm, 12mm, 13 mm, or 14 mm.

The second attachment axis may cross or be close to (distance less than 0.5 mm) the central axis in the first state of the drug delivery device.

The second attachment axis may be positioned at a second secondary distance from the central axis in the second state of the drug delivery device. The second secondary distance may be larger than 0.5 mm, such as in the range from 1 mm to 15 mm or larger than 1 mm, e.g. 2 mm, 3 mm, 4 mm, 5 mm, 6mm, 7 mm, 8mm, 9mm, 10mm, 11mm, 12mm, 13 mm, or 14 mm.

The second attachment axis may cross or be close to (distance less than 0.5 mm) the central axis in the second state of the drug delivery device.

In one or more exemplary drug delivery devices, the first attachment part (first attachment axis) and/or the second attachment part (second attachment axis) may be configured to be at an angle to each other when they intersect a plane that includes the central axis. The plane may be a plane that contains the central axis, where the plane also includes a radial axis extending at a right angle from the central axis. When the first attachment part intersects the plane, the first attachment axis of the first attachment part may be at an angle to the plane so that the first distal end of the first attachment part is the first part of the first attachment part that intersects the plane, where the remaining parts of the first attachment part intersects the plane subsequently during rotational movement. The second attachment part may intersect the same plane from the opposite side, where the second distal end of the second attachment part is the first part of the second attachment part that intersects the plane, where the remaining parts of the second attachment part intersects the plane subsequently during rotational movement. Thus, the second attachment part optionally intersects the plane from an opposing rotational direction than the first attachment part. This may also mean that when the first distal end and the second distal end of respective first attachment part and second attachment parts each contact the plane, the first attachment part (first attachment axis) is at an angle to the plane as well as at an angle to the second attachment part (second attachment axis). The angle between the first attachment part (first attachment axis) and the second attachment part (second attachment axis) to the plane may be approximately half the size of the angle between the first attachment part and the second attachment part.

In one or more exemplary drug delivery devices, the first body part may be configured to rotate in a first direction and the second body part may be configured rotate in a second direction, where the first direction is opposite to the second direction. Thus, as an example, the first body part may rotate in a clockwise direction, while the second body part may rotate in an opposed anti clockwise direction. In one or more examples where the drug delivery device comprises three or more body parts, abutting or neighbouring body parts may rotate in opposite directions. This may also mean that every second body part may rotate in the same direction. For example, where a first body part and a third body part rotate in the same first direction, then a second body part and/or a fourth body part may rotate in a second direction opposite the first direction.

In one or more exemplary drug delivery devices, the actuator mechanism may comprise one or more resilient parts, such as a plurality of resilient parts.

In one or more exemplary drug delivery devices, the first distal end of the first attachment part and/or the second distal end of the second attachment part may be provided with a sharp tip configured to penetrate a biological tissue. The sharp tip may be positioned in the vicinity of the distal end of the respective attachment part, where the sharp tip may be configured to have a diameter at the distal end which is smaller than a diameter of the attachment part at a distance from the distal end. The sharp tip may be configured in such a manner that when a rotational force is applied to the first attachment part, and a counterforce is applied to the second attachment part, the counterforce may cause the sharp tip to penetrate the biological tissue due to the force applied by the actuator mechanism.

When the first attachment part and/or the second attachment part penetrates the biological tissue due to the rotation between the first body part and the second body part (the first distal end moving towards the second distal end, the respective penetration point(s) in the biological tissue may be utilized to deliver a drug substance from the drug delivery device into the biological tissue, and where the drug substance may be introduced into the biological tissue that is beyond the mucous membrane. Thereby, the drug substance may enter the bloodstream more easily than if the drug substance is released in the stomach or intestinal lumen, and the drug delivery may be more effective. An example of this is when the drug substance is insulin, where insulin may degrade inside the gastrointestinal tract and is not capable of being absorbed from the gastrointestinal tract, but where a mucous membrane has been penetrated, and the insulin released through the penetrated gastrointestinal wall, the insulin will remain intact and reach the bloodstream of the user via the blood vessels in the intestinal layer beyond the mucous membrane (surface).

In one or more exemplary drug delivery devices, the first attachment part and/or the second attachment part may be provided with a gripping part configured to grip a biological tissue. The gripping part may be utilized to improve traction between the attachment part and a mucous membrane, allowing the attachment part to anchor the drug delivery device inside the body of the user. The gripping part may be a part that increases a mechanical friction between the attachment part and the surface to be attached to, where the gripping part may e.g. have a hook shape, or e.g. a shape where the gripping part of the first attachment part faces the gripping part of the second attachment part, so that the biological tissue which is positioned between the first attachment part and the second attachment part is gripped between the two gripping parts.

In one or more exemplary drug delivery devices, a part of the resilient part may be connected to the first body part and a second part of the resilient part may be connected to the second body part. This means that the resilient part may be utilized to store energy, such as rotational energy or rotational force which is applied to the first body part and the second body part, where the energy is stored in the resilient part. Furthermore, when the energy is released, e.g. when a locking element is dissolved or degraded, the force may be released to both the first body part and the second body part, which in turn transfers the force to the first attachment part and the second attachment part. The resilient part may e.g. be in the form of a helical spiral spring (mainspring) and/or a spiral torsion spring, where the first body part may be wound relative to the second body part by rotating the first body part relative to the second body part. This stores energy in the mainspring by twisting the spiral tighter. The stored force of the mainspring may then rotate the first body part in the opposing direction as the mainspring unwinds. Thus, the force of the mainspring may cause the first attachment part and the second attachment parts to travel in opposing directions, and where the attachment parts may pinch the biological tissue and either pinch the tissue or penetrate the tissue in order to attach the drug delivery device to the biological tissue.

When the drug delivery device has entered the body, and has e.g. entered the desired part in the gastrointestinal tract, the drug delivery device may be configured to transform from the first state to the second state. The transformation may be initiated by different means, where e.g. the first and the second body parts may be held in the first state using a locking mechanism e.g. comprising one or more locking elements made of a dissolvable, expandable or degradable material, where the material reacts with the surroundings, such as fluids, inside the desired body part, thereby unlocking or releasing the locking mechanism. The material of the locking elements may be a material that loses its structural force when in contact with the surroundings inside the desired body part. An example may be where locking element(s) is made of a polymeric material or a sugary substance which may dissolve, expand or degrade when it comes into contact with a certain kind of fluid which may include an enzyme or a certain kind of acid inside the digestive system. When the locking element comes into contact with the reagent, the material may dissolve, expand or degrade over time, and when the rotational force of the drug delivery device exceeds the static force of the locking element, the rotational force may be released via a rotation of the first body part relative to the second body part, or vice versa.

**In** one or more exemplary drug delivery devices, a locking element may fix an attachment part in a position where the attachment element locks the first body part in relation to the second part, i.e. prevents the first body part from rotating in relation to the second body part. When the locking element dissolves or degrades, the attachment part can move to a secondary position where the attachment part does not lock the first body part in relation to the second part, e.g. by the actuator mechanism causing a rotation of the attachment part about a rotation axis in relation to the body part to which the attachment part is rotationally attached.

The second state of the drug delivery device may be seen as the state which is initiated by the release of energy stored in the actuator mechanism, e.g. resilient part(s) of the actuator mechanism into a rotational force of the first and/or the second body part and/or a rotational force of the first attachment part in relation to the first body part. A termination of the second state may be seen as a point in time where the energy stored in the resilient part becomes stationary again, i.e. when the attachment parts have gripped or penetrated biological tissue and/or the rotational movement between the first body part and the second body part is stopped.

In one or more exemplary drug delivery devices, the drug delivery device may have a first state where the actuator mechanism has a constant resilient force load and a second state where the actuator mechanism releases the resilient force load. **In** the first state, the constant resilient force load may be seen as the energy stored in the actuator mechanism, and where the resilient force load is larger than zero. The second state may be seen as a state where the actuator mechanism releases its resilient force load, where the resilient force load is reduced, e.g. approaches zero, e.g. by rotating the first body part in relation to the second body part. The second state may be terminated when the attachment parts come into contact with or penetrates a biological tissue and the resilient force load does not change, even though it has not reached zero. Thus, a third state may follow the second state, when the drug delivery device has been attached to a wall of biological material, and the resilient force load is stationary after a resilient force release.

The first attachment part and/or the second attachment part may have an unfolding function, where during the first state of the drug delivery device, i.e. the initial state of the drug delivery device, the attachment parts are positioned or arranged inside the first and/or the second body part, or alternatively where the first and/or second attachment parts may be folded along the sides of the body parts. Other ways of obtaining the same may be envisioned. The folded state (first state) may e.g. be maintained using a releasable locking mechanism in the form of an encapsulation similar to a drug substance capsule, a band or plug, e.g. made of gelatine, sugars or other dissolvable materials or materials that lose their structural force. Thus, the attachment parts may be held in place until the drug delivery device has entered the gastrointestinal tract, e.g. the stomach, so that the attachment parts do not interfere or damage the lining of the mouth and/or the oesophagus. Prior to or during the transition to the second state the attachment parts may extend from the body parts and outwards, making the attachment parts ready to interact with a lining of the digestive system. When the attachment part or parts are in a folded or collapsed position, the distance from the central axis to the distal end of the attachment part being longer in the second state than in a first state. Thus, the diameter of the drug delivery device in the first state will be less in than the diameter of the drug delivery device in the second state.

In one or more exemplary drug delivery devices, at least part of the first attachment part and/or the second attachment part, such as the first needle and/or the second needle may be made of material comprising one or more of magnesium, titanium, iron and zinc which allows for accurate and precise control of the size and/or shape/geometry of the first attachment part and/or second attachment part in turn allowing for a delivery device with desired attachment capabilities and/or small production variances which is in particular important in the pharmaceutical industry.

The first attachment part, such as the first needle, may be made of material comprising one or more of magnesium, titanium, iron and zinc. The material of the first attachment part/first needle may be biocompatible and/or biodegradable such as a biocompatible material and/or a biodegradable material. The material of the first attachment part/first needle may comprise one or more biodegradable polymers such as PLA and/or POLGA. Some of, part of, most of, substantially all, or all of the material of the first attachment part/first needle may be biocompatible and/or biodegradable. The material of the first attachment part, such as the first needle, may comprise, consist of, or essentially consist of, biocompatible and/or biodegradable material such as biocompatible and/or biodegradable metals. The material of the first attachment part, such as the first needle, may comprise a biodegradable or bioresorbable metal or metal alloy, such as magnesium, zinc, and/or iron or an alloy comprising one or more of magnesium, zinc and iron. A biodegradable or bioresorbable metal or metal alloy may be understood as a metal or metal alloy that degrades safely within e.g. a human body in a practical amount of time, for example related to their application. The material of the first attachment part, such as the first needle, may comprise one or more metals such as a combination of one or more metals e.g. as a metal alloy.

The second attachment part, such as the second needle, may be made of material comprising one or more of magnesium, titanium, iron and zinc. The material of the second attachment part/second needle may be biocompatible and/or biodegradable such as a biocompatible material and/or a biodegradable material. The material of the second attachment part/second needle may comprise one or more biodegradable polymers such as PLA and/or POLGA. Some of, part of, most of, substantially all, or all of the material of the second attachment part/second needle may be biocompatible and/or biodegradable. The material of the second attachment part, such as the second needle, may comprise, consist of, or essentially consist of, biocompatible and/or biodegradable material such as biocompatible and/or biodegradable metals. The material of the second attachment part, such as the second needle, may comprise a biodegradable or bioresorbable metal or metal alloy, such as magnesium, zinc, and/or iron or an alloy comprising one or more of magnesium, zinc and iron. A biodegradable or bioresorbable metal or metal alloy may be understood as a metal or metal alloy that degrades safely within e.g. a human body in a practical amount of time, for example related to their application. The material of the second attachment part, such as the second needle, may comprise one or more metals such as a combination of one or more metals e.g. as a metal alloy.

An advantage of having a biodegradable material used in the attachment part(s) may be that the delivery device is able to deliver an active drug substance or payload arranged in the attachment part(s) and/or body parts of the delivery device at a specific part of the body of the subject, e.g. such as the stomach or intestines after the delivery device has attached to the internal surface, e.g. the intestinal wall, thanks to the sharp properties of the material of the attachment part(s), and for an extended period of time, since the biodegradable material will degrade gradually in time. Further, when the material of the attachment part(s) is biodegradable, the attachment part(s) will degrade in the human body and disappear after having delivered the payload/active drug substance comprised in the drug delivery device, thereby avoiding harming the human subject over time. The attachment part(s) may be configured to degrade in a period of time of hours, e.g. 2 hours, 5 hours, 10 hours, 20 hours, or 24 hours, days, e.g. 1 day, 2 days, 5 days, or weeks, e.g. 1 week, 2 weeks, 3 weeks, or 5 weeks.

The material of the attachment part(s), such as the needle(s), may comprise one or more or a combination of magnesium (Mg), zinc (Zn), and/or iron (Fe). An advantage of having the attachment part(s) of a material comprising Mg, Zn, and/or Fe may be that the shape and size of the attachment part(s) can be precisely controlled thereby providing improved attachment to the internal surface, for example to an internal wall of the intestines of the human subject.

For example, the material of the attachment part(s), such as the needle(s), may comprise 0,001 wt% to 100wt% of biodegradable metal such as 0,001wt% to 100wt% of magnesium, 0,001wt% to 100wt% of zinc, 0,001wt% to 100wt% of iron.

The material of the attachment part(s), such as the needle(s), may for example comprise 0,001 wt% of Mg, 0,005 wt% of Mg, 0,01 wt% of Mg, 0,05 wt% of Mg, 0,1 wt% of Mg, 0,5 wt% of Mg , 1 wt% of Mg, 5 wt% of Mg, 10 wt% of Mg, 20 wt% of Mg, 30 wt% of Mg, 40 wt% of Mg, 50 wt% of Mg, 60 wt% of Mg, 70 wt% of Mg, 80 wt% of Mg, 90 wt% of Mg, or 100 wt% of Mg.

The material of the attachment part(s), such as the needle(s), may for example comprise 0,001 wt% of Zn, 0,005 wt% of Zn, 0,01 wt% of Zn, 0,05 wt% of Zn, 0,1 wt% of Zn, 0,5 wt% of Zn , 1 wt% of Zn, 5 wt% of Zn, 10 wt% of Zn, 20 wt% of Zn, 30 wt% of Zn, 40 wt% of Zn, 50 wt% of Zn, 60 wt% of Zn, 70 wt% of Zn, 80 wt% of Zn, 90 wt% of Zn, or 100 wt% of Zn.

The material of the attachment part(s), such as the needle(s), may for example comprise 0,001 wt% of Fe, 0,005 wt% of Fe, 0,01 wt% of Fe, 0,05 wt% of Fe, 0,1 wt% of Fe, 0,5 wt% of Fe , 1 wt% of Fe, 5 wt% of Fe, 10 wt% of Fe, 20 wt% of Fe, 30 wt% of Fe, 40 wt% of Fe, 50 wt% of Fe, 60 wt% of Fe, 70 wt% of Fe, 80 wt% of Fe, 90 wt% of Fe, or 100 wt% of Fe.

The material of the attachment part(s), such as the needle(s), may comprise a metal alloy such as Zn-Mg, Zn-Fe, Mg-Fe, or Zn-Mg-Fe. The material of the attachment part(s), such as the needle(s), may for example comprise an alloy of Zn-Mg with 0,001 wt% of Mg, 0,005 wt% of Mg, 0,01 wt% of Mg, 0,05 wt% of Mg, 0,1 wt% of Mg, 0,5 wt% of Mg , 1 wt% of Mg, 5 wt% of Mg, 10 wt% of Mg, 20 wt% of Mg, 30 wt% of Mg, 40 wt% of Mg, 50 wt% of Mg, 60 wt% of Mg, 70 wt% of Mg, 80 wt% of Mg, or 90 wt% of Mg.

The material of the attachment part(s), such as the needle(s), may for example comprise an alloy of Zn-Fe with 0,001 wt% of Fe, 0,005 wt% of Fe, 0,01 wt% of Fe, 0,05 wt% of Fe, 0,1 wt% of Fe, 0,5 wt% of Fe , 1 wt% of Fe, 5 wt% of Fe, 10 wt% of Fe, 20 wt% of Fe, 30 wt% of Fe, 40 wt% of Fe, 50 wt% of Fe, 60 wt% of Fe, 70 wt% of Fe, 80 wt% of Fe, or 90 wt% of Fe.

The material of the attachment part(s), such as the needle(s), may for example comprise an alloy of Mg-Fe with 0,001 wt% of Fe, 0,005 wt% of Fe, 0,01 wt% of Fe, 0,05 wt% of Fe, 0,1 wt% of Fe, 0,5 wt% of Fe , 1 wt% of Fe, 5 wt% of Fe, 10 wt% of Fe, 20 wt% of Fe, 30 wt% of Fe, 40 wt% of Fe, 50 wt% of Fe, 60 wt% of Fe, 70 wt% of Fe, 80 wt% of Fe, or 90 wt% of Fe.

The material of the attachment part(s), such as the needle(s), may for example comprise an alloy of Zn-Mg-Fe with 0,001 wt% of Fe, 0,005 wt% of Fe, 0,01 wt% of Fe, 0,05 wt% of Fe, 0,1 wt% of Fe, 0,5 wt% of Fe , 1 wt% of Fe, 5 wt% of Fe, 10 wt% of Fe, 20 wt% of Fe, 30 wt% of Fe, 40 wt% of Fe, 50 wt% of Fe, 60 wt% of Fe, 70 wt% of Fe, 80 wt% of Fe, 90 wt% of Fe, 0,001 wt% of Mg, 0,005 wt% of Mg, 0,01 wt% of Mg, 0,05 wt% of Mg, 0,1 wt% of Mg, 0,5 wt% of Mg , 1 wt% of Mg, 5 wt% of Mg, 10 wt% of Mg, 20 wt% of Mg, 30 wt% of Mg, 40 wt% of Mg, 50 wt% of Mg, 60 wt% of Mg, 70 wt% of Mg, 80 wt% of Mg, 90 wt% of Mg, 0,001 wt% of Zn, 0,005 wt% of Zn, 0,01 wt% of Zn, 0,05 wt% of Zn, 0,1 wt% of Zn, 0,5 wt% of Zn , 1 wt% of Zn, 5 wt% of Zn, 10 wt% of Zn, 20 wt% of Zn, 30 wt% of Zn, 40 wt% of Zn, 50 wt% of Zn, 60 wt% of Zn, 70 wt% of Zn, 80 wt% of Zn, or 90 wt% of Zn.

The attachment part(s), such as the needle(s), may be made of a material comprising one or more thermoplastic or thermoset polymers. The material of the attachment part(s), such as the needle(s), may comprise one or more active drug substances. Thus, an active drug substance may be embedded in the material of the attachment part(s), such as the needle(s), to form a pharmaceutical composition.

In some embodiments, the attachment part(s), such as the needle(s), may comprise for example water soluble, water insoluble, biodegradable, non-biodegradable and/or pH dependent soluble materials. In some embodiments, the attachment part(s), such as the needle(s), may comprise a water soluble, biodegradable and/or pH-dependent material that may dissolve and/or degrade so that the attachment part(s), such as the needle(s), lodged in the intestinal tissue may gradually degrade and/or dissolve. In some embodiments, the attachment part(s), such as the needle(s), may comprise a watersoluble material to allow immediate release or modified release of the active drug substance depending of the material selected. In some embodiments, a water insoluble or biodegradable material may allow depot of the active drug substance in the attachment part(s), such as the needle(s), for longer release duration (for example days, weeks or months). In some embodiments, a pH dependent soluble material may allow the attachment part(s), such as the needle(s), to stay intact at pH conditions below the physiologic for example a pH of approximately 7.4 to remain intact in the gastrointestinal lumen, but then may dissolve once inside the gastrointestinal wall. In some embodiments, one or more water soluble, water insoluble, biodegradable and/or pH dependent materials may optionally be combined to control release of the active drug substance for example by diffusion or erosion of the attachment part(s), such as the needle(s), for controlled release duration (for example minutes, hours, days, weeks, or months).

In some embodiments, the attachment part(s), such as the needle(s), may be made from different compositions. For example, an outer part of the attachment part(s), such as the needle(s),may be made of one composition and an inner core of the attachment part(s), such as the needle(s), may be made from another composition. In some embodiments, the outer part and the inner core of the attachment part(s), such as the needle(s), may be composed of for example a water soluble, a water insoluble, a biodegradable, and/or a pH dependent material. In some embodiments, one or more water soluble, water insoluble, biodegradable and/or pH dependent materials may be combined to control the release of the active drug substance once the attachment part(s), such as the needle(s), may move its position from the lumen to the internal tissue for example the gastrointestinal lumen to the gastrointestinal tissue.

In some embodiments, the attachment part(s), such as the needle(s), may be tubular and may include a tubular body and the tubular body may comprise an active drug substance for example a liquid payload comprising the active drug substance, optionally connected to a tubular attachment part so the payload with the active drug substance may flow though the attachment part(s), such as the needle(s), into the internal tissue for example the intestinal tissue. In some embodiments, the tubular body may contain expandable such as swelling excipients that may expand by a chemical reaction for example when mixed expand in volume and/or produce a gas to advance the delivery of the payload. In some embodiments, the expansion is by osmosis.

In some embodiments, the first compartment (compartment to hold active drug substance) may comprise a closure part for closing the first compartment. The closure part may contribute to improved control of release of the active drug substance. In some embodiments, the closure part may be composed of for example a water soluble, a water insoluble, a biodegradable, and/or a pH dependent material. In some embodiments, one or more water soluble, water insoluble, biodegradable and/or pH dependent materials may be combined to control the release of the active drug substance from the first compartment once the attachment part(s), such as the needle(s), moves its position from the lumen to the internal tissue for example from the gastrointestinal lumen to the gastrointestinal tissue.

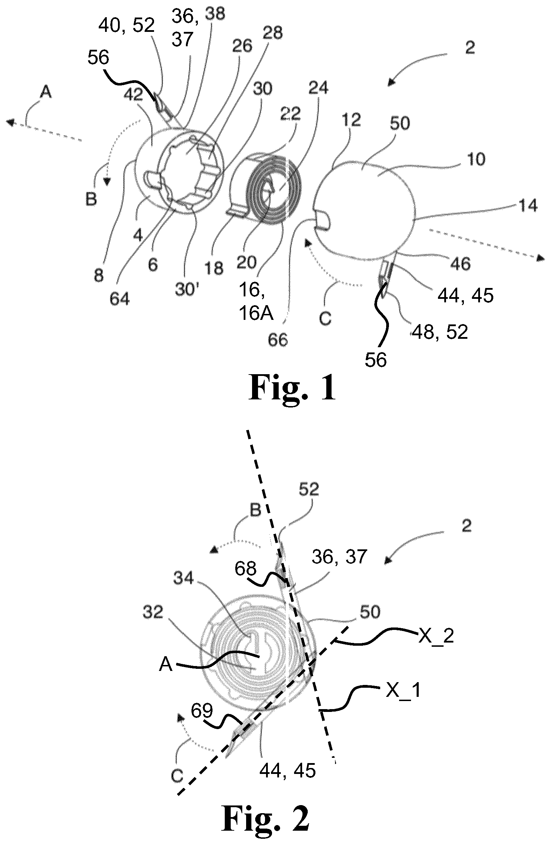

Fig. 1 shows an exploded view of a drug delivery device 2 in accordance with the disclosure, where the drug delivery device comprises a first body part 4, having a first end 6 and a second end 8, a second body part 10 having a first end 12 and a second end 14. When assembled, the first body part 4 is rotatably connected to the second body part 10, where the first end 6 of the first body part abuts the first end 12 of the second body part when connected.

The drug delivery device 2 further comprises an actuator mechanism 16 comprising a resilient part 16A, in this example in the form of a spiral torsion spring. A first part 18 of the resilient part 16A (first end of spiral torsion spring) is positioned on an outer periphery 22 of the spiral torsion spring, and a second part 20 of the resilient part 16A (second end of spiral torsion spring) is in a center part 24 of the spiral torsion spring.

The first body part 4 comprises an inner volume 26, where the inner volume is adapted to receive the resilient part 16A, and where an inner surface 28 of the inner volume 26 comprises one or more first engagement parts 30 which are configured to engage with the first part 18 of the resilient part 16A, and where the first engagement parts can maintain the position of the first part during rotational movement of the first body part 4 and the second body part 10 relative to each other. The second part 20 of the resilient part 16A is configured to engage with a second engagement part 32 (see Fig. 2) which is positioned centrally inside the second body part 10. The second engagement part 32, as seen in Fig. 2, is configured to extend into the central part 24 of the spring, when the spring is positioned inside the inner volume 26 of the first body part 4. The second engagement part 32 comprises a slit or a groove 34 which is adapted to engage with the second part 20 of the resilient part 16A, so that a rotational movement of the first 4 and/or the second body part 10 can wind up the resilient part 16A, when the first part 18 is in engagement with the first engagement part 30.

The drug delivery device 2 has a central axis A, which extends in a direction from the second end 8 of the first body part 4 (first end of drug delivery device) towards the second end 14 of the second body part (second end of drug delivery device). The central axis A may be seen as defining the primary axis about which the first body part 4 and the second body part 10 rotates.

The first engagement part 30 and the first part 18 of the resilient part 16A may have an engagement which means that when the load in the spring exceeds a predefined level, the first end releases the first engagement part 30, and jumps into engagement with the next engagement part 30'. This means that the drug delivery device may have a torque limiter, where the torque limiter ensures that the stored energy inside the resilient member 16 cannot exceed a predefined limit.

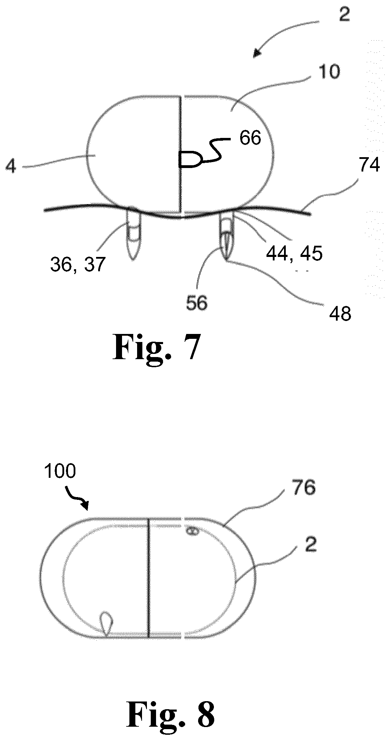

The drug delivery device 2 comprises a first attachment part 36 having a first proximal end 38 and a first distal end 40. The first attachment part 36 comprises a straight first needle 37 and is fixedly attached to the first body part 4. The first attachment part 36 extends along a first attachment axis, see Fig. 2, from an outer surface 42 of the first body part 4 in a direction away from the outer surface 42. The first distal end 40 of the first attachment part 36 may be a sharp tip in order to allow penetration of biological tissue, where the rotational force provided by the resilient member 16A may be used to penetrate a bodily tissue, see also Fig. 7.

The drug delivery device 2 comprises a second attachment part 44 having a second proximal end 46 and a second distal end 48. The second attachment part 44 comprises a straight second needle 45 and is fixedly attached to the first body part 4. The second attachment part 44 extends along a second attachment axis, see Fig. 2, from an outer surface 50 of the second body part 10 in a direction away from the outer surface 50. The second distal end 48 of the second attachment part 44 may be a sharp tip in order to allow penetration of biological tissue, where the rotational force provided by the resilient member 16A may be used to penetrate a bodily tissue, see also Fig. 7.

The distal ends 40, 48 of attachment parts 36, 44 may be a sharp tip 52, where the sharp tip 52 may be similar to a sharp tip of a hypodermic needle, where the sharp tip 52 is capable of penetrating bodily tissue, such as a mucous membrane of the intestine, stomach, bowels, or other parts of the digestive system and/or the gastrointestinal system. The needles 37,45 may be hollow with an opening 56 at the distal ends 40, 48, so that an active drug substance may be introduced via the opening 56 into a bodily tissue after the attachment parts 36, 44 have penetrated the biological tissue, as seen in Fig. 7.

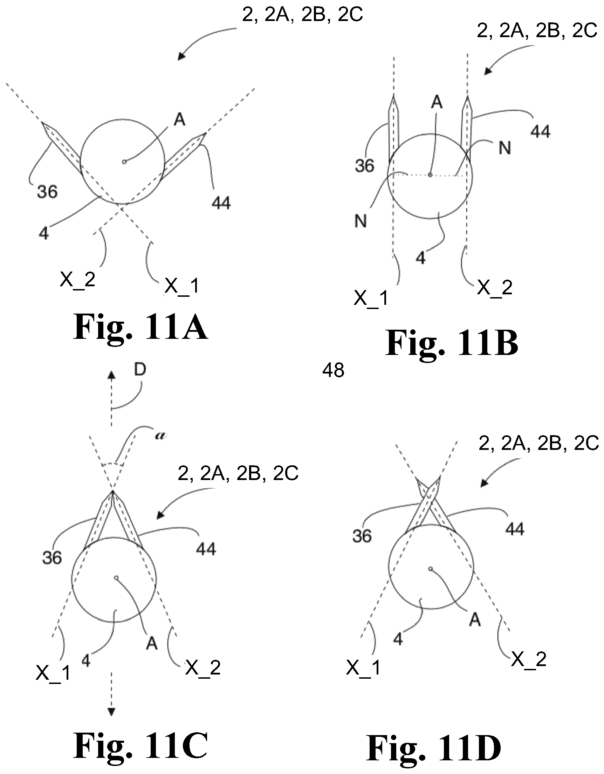

The resilient force of the resilient part 16A is used to rotate the first body part in a first direction B and the second body part in a second direction C about the central axis A being the primary axis, as seen in Fig. 1 and 2, and shown in more detail in Fig. 11a - 11. In other words, the actuator mechanism 16 (resilient part 16A) is configured to move the first distal end 40 towards the second distal end 48.

The first body part 4 has a first primary recess 64 in the outer surface 42 and the second body part 10 has a second primary recess 66 in the outer surface 50. The first primary recess 64 and the second primary recess 66 are part of a locking mechanism for locking, e.g. preventing rotation of, the first body part 4 in relation to the second body part 10 when the drug delivery device 2 is in the first state by arranging a first locking element in the first primary recess 64 and the second primary recess 66.

Fig. 2 shows a sectional side view of drug delivery device 2. The first attachment part 36/ first needle 37 extends along first attachment axis X_1 perpendicular to the central axis A. The second attachment part 44/second needle 45 extends along second attachment axis X_2 perpendicular to the central axis A. The first needle 37 optionally comprises a first compartment 68 configured to accommodate an active drug substance. The first compartment 68 is optionally formed as a through-going bore or a cavity in the first needle 37. The second needle 45 optionally comprises a second compartment 69 configured to accommodate an active drug substance. The second compartment 69 is optionally formed as a through-going bore or a cavity in the second needle 45.

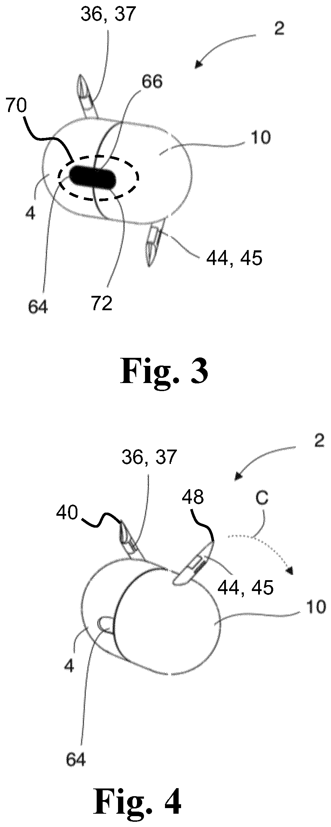

Fig. 3 shows drug delivery device 2 in a first state. The drug delivery device comprises a locking mechanism indicated with dotted oval 70 comprising first primary recess 64, second primary recess 66 and a first locking element 72 arranged in the first primary recess 64 and the second primary recess 66. The first locking element 72 is capable of preventing rotational movement of the first body part 4 and the second body part 10 relative to each other, to maintain a static relationship between the body parts 4, 10. The first locking element 72 may be in the form of a degradable material, such as a sugary substance, where a contact with the fluids of the gastrointestinal tract leads to the degradation of the first locking element 72 material. When the rotational force applied to the body parts 4, 10 via the resilient member 16A exceeds the static force of the (degraded) first locking element 72, the first locking element 72 will release the body parts 4, 10, and allow the resilient member 16A to discharge of its stored energy, causing the rotation of the first body part 4 relative to the second body part 10 in a second state of the drug delivery device.

Fig. 4 shows the drug delivery device 2, where the first locking element 72 has been degraded or dissolved, and the second body part 10 has rotated in the direction C relative to the first body part 4. Thus, the second attachment member 44, second distal end 48 has moved from a second primary position in the first state as in Fig. 3 to a second secondary position seen in Fig. 4, via the rotational force (torque) applied to the body parts 4, 10 from inside the inner volume 26. During the rotation of the first body part 4 in relation to the second body part 10, the actuator mechanism has moved the first distal end 40 towards the second distal end 48.

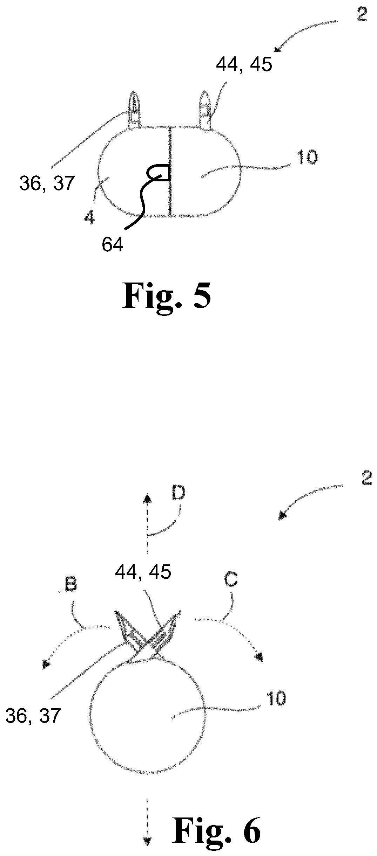

Fig. 5 and Fig. 6 show drug delivery device 2 seen in Fig. 4 from a side view and end view, where it may be seen that the attachment members 36, 44 have passed an axis D, which may e.g. be seen as a plane which includes both the central axis A and the axis D. When the attachment members 36, 44 pass the imaginary plane (seen as axis D) the distal ends of the attachment members and the opposing force applied in direction B for the first attachment part 36 and direction C for the second attachment part 44, can ensure that the distal ends can grip a surface area, and either penetrate or grip the surface of the biological tissue. When the drug delivery device 2 is e.g. inside the intestine, the intestine will push the device up to at least one surface area of biological tissue, so that the force applied to the attachment parts will not push the device away from the surface, as the opposing surface will hold the device close to a surface. If the device does not grip in a first instance, the actuator mechanism may have enough force for a plurality of rotations, so that when the attachment parts are near each other again, the attachment parts will again try to grip the surface and fix the drug delivery device relative to the biological tissue.

Fig. 7 shows drug delivery device 2 in second state after attachment to bodily tissue 74 such as a stomach wall or intestine wall. The rotation of the first body part 4 in relation to the second body part 10 and the movement of the first distal end 40 towards the second distal end 48 has resulted in the distal ends 40, 48 having penetrated the bodily tissue 74 to be inside the bodily tissue. The remaining resilient force from the actuator mechanism maintains the attachment parts 36, 44 inside the bodily tissue 74. Thus, the drug delivery device 2 is attached to the bodily tissue, and active drug substance may be released from compartments 68, 69 and/or via opening 56 e.g. to reach the blood stream via the blood vessels of the bodily tissue 74.

Fig. 8 shows an exemplary pharmaceutical composition 100 comprising drug delivery device 2 where the drug delivery device 2 is encapsulated in a housing 76 optionally made of dissolvable material. The pharmaceutical composition 100 comprises active drug substance arranged in first compartment 68 and/or second compartment 69. The housing 76 may enclose the drug delivery device 2 to make it easier to swallow. The dissolvable housing 76 may dissolve inside the gastrointestinal tract, and where the drug delivery device 2 cannot engage or attach before the housing 76 is dissolved. These kinds of housings are known in the art, in the form of a drug capsule, where the material of drug capsule may e.g. be a gelatin, similar to hard drug capsule shells known in the art. **In** one or more exemplary pharmaceutical compositions, the drug delivery device may be coated with a coating.

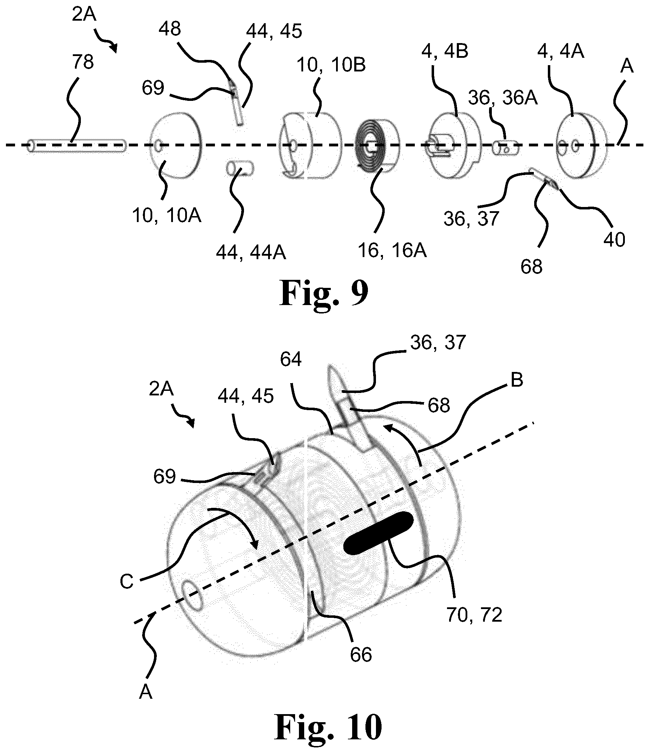

Fig. 9 shows an exploded view of an exemplary drug delivery device according to the present disclosure. The drug delivery device 2A has a central axis A and comprises a two-part first body part 4 comprising first primary body part 4A and first secondary body part 4B. The drug delivery device 2A comprises a first attachment part 36 comprising a first base 36A and first needle 37 attached to the first base 36A. The first attachment part 36 has a first distal end 40 and is rotationally attached to the first body part 4 via a first joint connection formed by cylindrical first base 36A and corresponding cylindrical cavity in the first body part 4, the first joint having a first rotation axis X_R_1. Thus, the first attachment part 36 is configured to rotate about first rotation axis in relation to the first body part 4. The first rotation axis X_R_1 is parallel to the central axis A.

The drug delivery device 2A comprises a two-part second body part 10 comprising second primary body part 10A and second secondary body part 10B. The drug delivery device 2A comprises a second attachment part 44 comprising a second base 44A and second needle 45 attached to the second base 44A. The second attachment part 44 has a second distal end 48 and is optionally rotationally attached to the second body part 10 via a second joint connection formed by cylindrical second base 44A and corresponding cylindrical cavity in the second body part 10, the second joint having a second rotation axis X_R_2. Thus, the second attachment part 44 is configured to rotate about first rotation axis in relation to the second body part 4. The second rotation axis X_R_2 is parallel to the central axis A.

The drug delivery device 2A comprises a frame part 78 formed as an axle member or rod, where different parts, such as the first body part and/or the second body part are attached, e.g. fixed or rotatably attached to the frame part 78.

The drug delivery device 2A comprises actuator mechanism 16 comprising resilient part 16A configured to move the first distal end 40 towards the second distal end 48 by rotating the first body part 4 in relation to the second body part 10.

Now referring to Fig. 10, the first attachment part 36 is configured to rotate about the first rotation axis in relation to the first body part 4 to move the first distal end 40 from a first primary position, e.g. in the first state, with a first primary radial distance from the central axis A of the delivery device 2A to a first secondary position (shown in Fig. 10) with a first secondary radial distance from the central axis A, wherein the first secondary radial distance is larger, such as at least 2 mm larger, than the first primary radial distance. The first body 4 comprises a first primary recess 64 accommodating the first attachment part 36 or at least parts thereof, e.g. in the first state. The actuator mechanism 16 is optionally configured to move the first distal end from the first primary position to the first secondary position. **In** the first secondary position, the first attachment part 36

The first attachment part 36 is configured to rotate about the first rotation axis in relation to the first body part 4 to move the first distal end from a first primary angular position of the first primary position to a first secondary angular position (shown in Fig. 10) of first secondary position in relation to a first proximal end of the first attachment part. **In** the illustrated drug delivery device 2A, an angle between the first primary angular position and the first secondary angular position is larger than 10° and even larger than 30°, such as in the range from 35° to 85°. The actuator mechanism 16 is optionally configured to move the first distal end from the first primary angular position to the first secondary angular position.

The second attachment part 44 is configured to rotate about the second rotation axis in relation to the second body part 10 to move the second distal end 48 from a second primary position, e.g. in the first state, with a second primary radial distance from the central axis A of the delivery device 2A to a second secondary position (shown in Fig. 10) with a second secondary radial distance from the central axis A, wherein the second secondary radial distance is larger, such as at least 2 mm larger, than the second primary radial distance. The second body 10 comprises a second primary recess 66 accommodating the second attachment part 44 or at least parts thereof, e.g. in the first state. The actuator mechanism 16 is optionally configured to move the second distal end from the second primary position to the second secondary position.

The second attachment part 44 is configured to rotate about the second rotation axis in relation to the second body part 10 to move the second distal end from a second primary angular position of the second primary position to a second secondary angular position (shown in Fig. 10) of second secondary position in relation to a second proximal end of the second attachment part. In the illustrated drug delivery device 2A, an angle between the second primary angular position and the second secondary angular position is larger than 10° and even larger than 30°, such as in the range from 35° to 85°. The actuator mechanism 16 is optionally configured to move the second distal end from the second primary angular position to the second secondary angular position.

Figs. 11A-11D show a schematic view of drug delivery devices 2, 2A, 2B, 2C where the first attachment part 36 and the second attachment part 44 are in different positions in the second state of the drug delivery device. The first attachment part 36 has a first attachment axis X_1 and the second attachment part 44 has a second attachment axis X_2. When the first attachment part 36 and the second attachment part 44 come into contact with a plane containing the central axis A and the plane axis D, the angle α, between the first attachment axis X_1 and the second attachment axis X_2 may be in the range from 5° to 75°, such as in the range from 20° to 60°. The size of the angle may increase or decrease with the distance between the central axis and the attachment axes X_1 and X_2, or when the length of the attachment members 36, 44 are changed.

However, the angle α ensures that in the transition from the position seen in Fig. 11A towards the position seen in Fig. 11C biological tissue may be pinched between the two attachment parts 36, 44 and if the attachment parts penetrate, the increased rotation towards the position shown in Fig. 11D pulls the drug delivery device 2 closer to a tissue surface that may be caught by the attachment parts, using the resilient force.

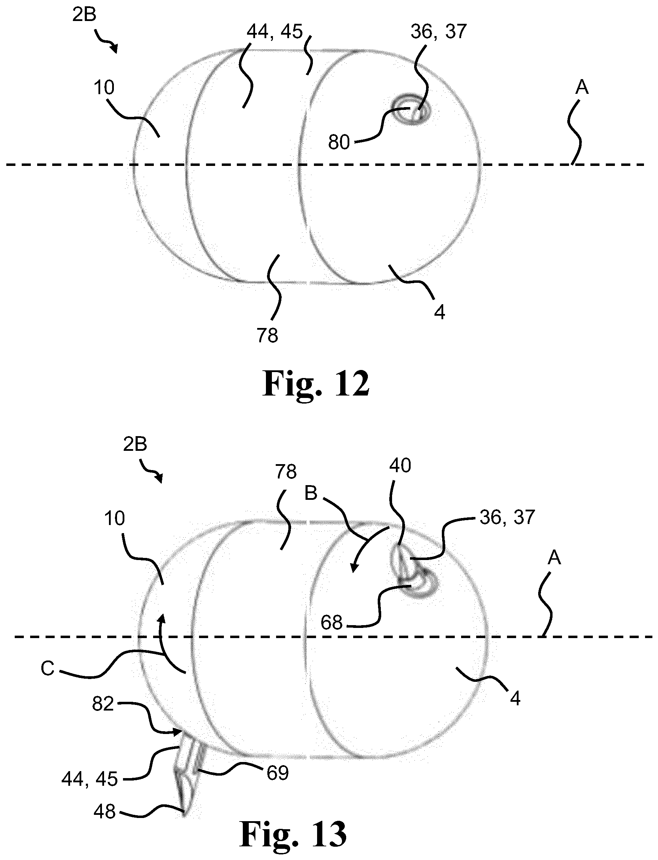

Fig. 12 shows an exemplary drug delivery device 2B in a first state and Fig. 13 shows the drug delivery device 2B in a second state. **In** the first state, the first attachment part 36 with the first needle 37 is optionally arranged inside the first body part 4 and/or with the first distal end in a first primary position with a first primary radial distance to the central axis. The first primary radial distance may be less than 10 mm, such as less than 8 mm or even less than 5 mm. In the first state, the first distal end 40 is optionally arranged inside the first body part 4.

In the first state, the second attachment part 44 with the second needle 45 is optionally arranged inside the second body part 10 and/or with the second distal end in a second primary position with a second primary radial distance to the central axis. The second primary radial distance may be less than 10 mm, such as less than 8 mm or even less than 5 mm. In the first state, the second distal end 48 is optionally arranged inside the second body part 10. Arranging the attachment part(s)/distal end(s) inside the body part(s) facilitates or allows for a smooth oral administration.

In the second state, the first distal end 40 has been ejected from the first body part 4 through first opening 80 in the first body part 4 to be in a first secondary position with a first secondary radial distance to the central axis. The first secondary radial distance is larger than the first primary radial distance and may be larger than 5 mm, such as larger than 6 mm, or larger than 8 mm.

In the second state, the second distal end 48 has been ejected from the second body part 10 through second opening 82 in the second body part 10 to be in a second secondary position with a second secondary radial distance to the central axis. The second secondary radial distance is larger than the second primary radial distance and may be larger than 5 mm, such as larger than 6 mm, or larger than 8 mm. **In** the second state of the drug delivery device 2B as shown in Fig. 13, the actuator mechanism (not shown) rotates the first body part 4 in relation to the second body part 10 and optionally in relation to frame part 78 about the central axis A to move the first distal end 40 towards the second distal end 48. The actuator mechanism may be configured to rotate the second body part 10 in relation to the frame part 78 about the central axis A.

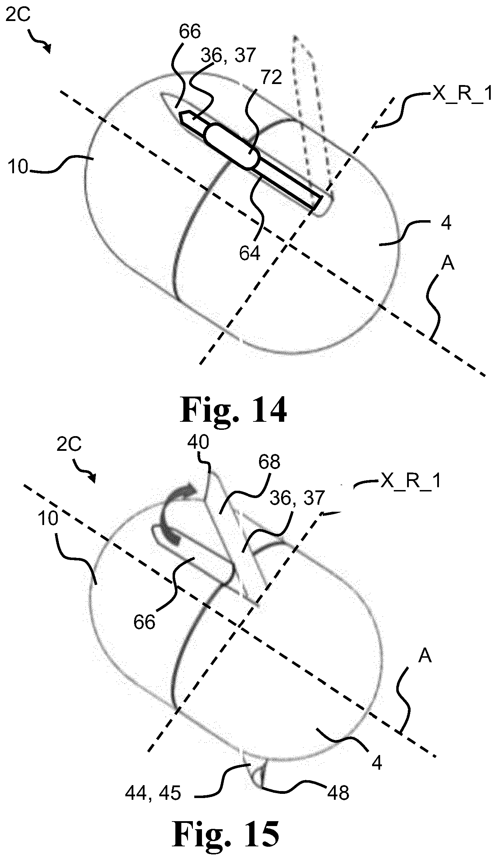

Fig. 14 shows an exemplary drug delivery device 2C in a first state and Fig. 15 shows the drug delivery device 2C in a second state. In the first state, the first attachment part 36 with the first needle 37 is arranged within first primary recess 64 and second primary recess 66 and locked by first locking element 72. Thus, the first attachment part 36 and the first locking element 72 prevents rotation of first body part and second body part. The second attachment part 44 is likewise arranged within first secondary recess of first body part and second secondary recess of second body part on the opposite side and locked with second locking element. In the first state, the first attachment axis of first attachment part 36 is substantially parallel to the central axis and the second attachment axis of the second attachment part is substantially parallel to the central axis.

The first locking element 72 is dissolved in the gastrointestinal tract and the first distal end 40 moves from its first primary position in the first state (Fig. 14) to a first secondary position in the second state (Fig. 15) by rotation about a first rotation axis X_R_1 perpendicular to the central axis A.

Likewise, the second locking element is dissolved in the gastrointestinal tract and the second distal end 48 moves from its second primary position in the first state (Fig. 14) to a second secondary position in the second state (Fig. 15) by rotation about a second rotation axis (not shown) perpendicular to the central axis A.

The angle between the first primary direction in the first state and the first secondary direction in the second state is at least 30°, such as 45° or more. The first secondary direction of the first attachment part may be perpendicular or substantially perpendicular to the central axis.

The angle between the second primary direction in the first state and the second secondary direction in the second state is at least 30°, such as 45° or more. The second secondary direction of the second attachment part may be perpendicular or substantially perpendicular to the central axis A. In the second state, the actuator mechanism moves the first distal end 40 towards the second distal end 48 by rotating the first body part 4 in relation to the second body part 10 thereby reducing the angle between the first secondary direction and the second secondary direction.

In the first state, the first attachment part 36 with the first needle 37 is arranged with the first distal end in a first primary position with a first primary radial distance to the central axis. The first primary radial distance may be less than 10 mm, such as in the range from 3 mm to 8 mm.

In the first state, the second attachment part 44 with the second needle 45 is arranged with the second distal end in a second primary position with a second primary radial distance to the central axis. The second primary radial distance may be less than 10 mm, such as in the range from 3 mm to 8 mm.

In the second state, the attachment parts 36, 44 have been unfolded from the body parts 4, 10 for the distal ends 40, 48 to be in a respective first secondary position with a first secondary radial distance to the central axis and a second secondary position with a second secondary radial distance to the central axis. The first secondary radial distance is larger than the first primary radial distance and may be larger than 5 mm, such as larger than 6 mm, or larger than 8 mm. The second secondary radial distance is larger than the second primary radial distance and may be larger than 5 mm, such as larger than 6 mm, or larger than 8 mm.

In the second state of the drug delivery device 2C as shown in Fig. 15, the actuator mechanism (not shown) rotates the first body part 4 in relation to the second body part 10 about the central axis A to move the first distal end 40 towards the second distal end 48.

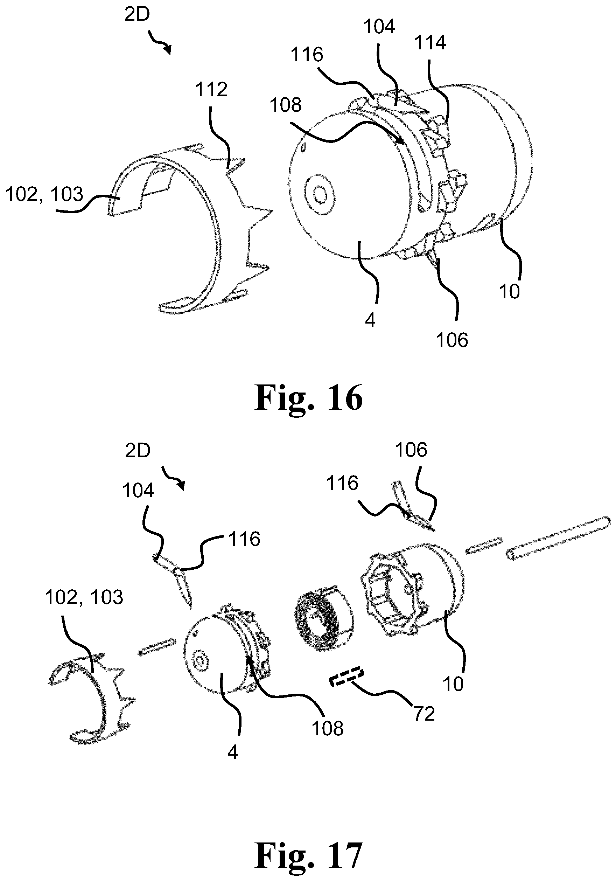

Fig. 16 shows an exemplary drug delivery device 2D, with Fig. 17 showing an exploded version of the exemplary drug delivery device 2D. The drug delivery device 2D may include any and/or all of the features discussed above with respect to Figs. 1-15 unless otherwise noted.

As shown, the drug delivery device 2D can include a first body recess 108 configured to allow rotation of the first attachment part 104. Further, the drug delivery device 2D can include a second body recess (not shown) configured to allow rotation of the second attachment part 106. The first attachment part 104 and the second attachment part 106 can both include a joint 116, thereby forming a bent needle or spike. This can allow for easier penetration of tissue.

Additionally, as shown the drug delivery system 2D can include a first locking band 102. The first locking band 102 can prevent rotation of the first body part 4 with respect to the second body part 10. First locking band 102 can be used instead of a locking element 72. Alternatively, the first locking band 102 can act as a first cover band 103 and be used in conjunction with a locking element 72. Specifically, the first locking band 102 can include a plurality of locking protrusions 112. The locking protrusions 112 can fit within the mating features 114 of the drug delivery system 2D. Once mated, the locking protrusions 112 prevent rotation of the first body part 4 and the second body part 10. The first locking band 112 can then dissolve to allow rotation.

Embodiments of the drug delivery device disclosed herein were used in animal studies which achieved the following experimental results. These experimental results illustrate the success of one or more exemplary drug delivery devices in actual use. Successful "hooking" (e.g., attachment) in the experimental results can be defined as being attached for at least 4 hours.

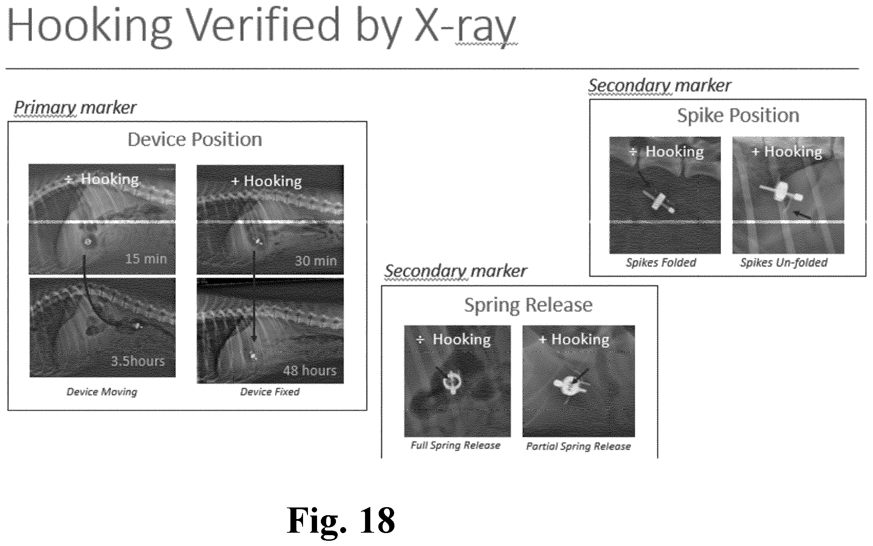

Fig. 18 shows x-ray images of the drug delivery device having hooked (e.g., attached, connected) via one or more of the first or second attachment part.

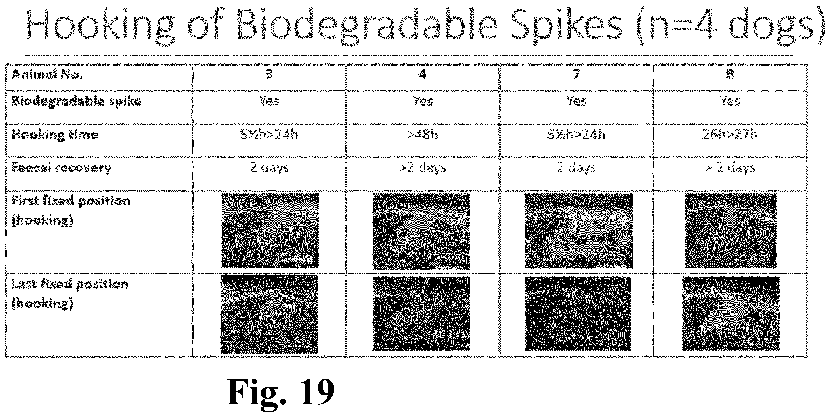

Fig. 19B shows x-ray images and further data on embodiments of the disclosed drug delivery device having biodegradable attachment parts. As shown, the first or second attachment parts have degraded in each of the animals tested, allowing for recovery of the drug delivery device. Further, as disclosed, the drug delivery device can be attached within a body for greater than 24 or 48 hours. All tests of the drug delivery device attached within tissue of the tested animal.

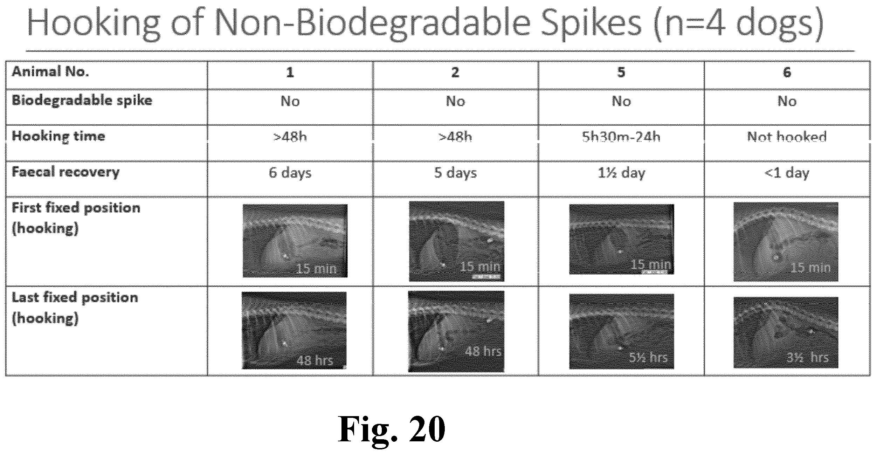

Fig. 20 shows further x-ray images and further data on embodiments of the disclosed drug delivery device having a non-biodegradable attachment part. This data shows further proof of the success of the drug delivery device, where the drug delivery device is attached for at least 24 or 48 hours. All but one of the drug delivery devices tested attached within tissue of the animal.

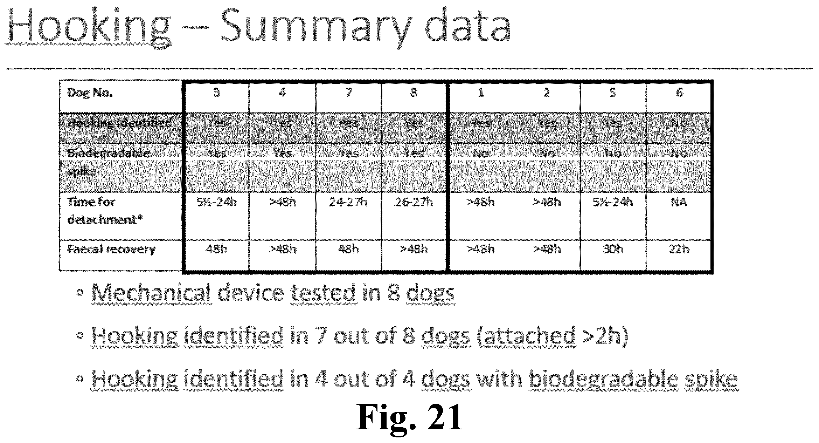

Fig. 21 summarizes data achieved using embodiments of the disclosed drug delivery devices.

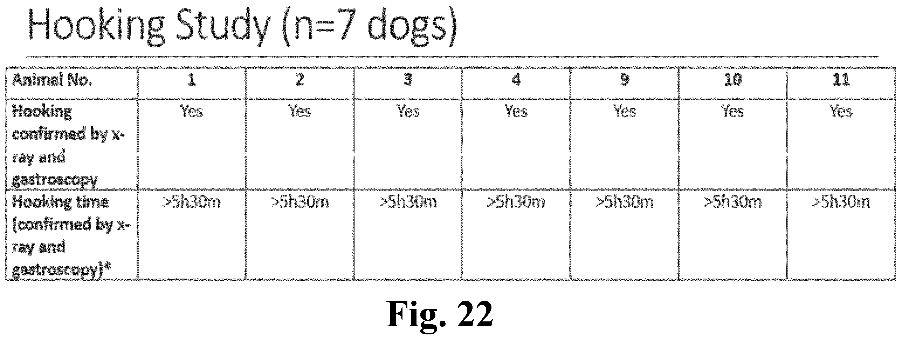

Fig. 22 illustrates a hooking study of embodiments of the disclosed drug delivery devices. As shown, all devices were confirmed to have hooked within tissue of the tested animal. Further, the devices remained attached within the tissue for at least 5 hours and 30 minutes.

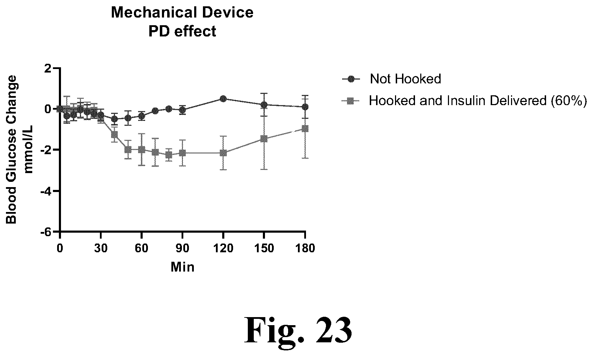

Fig. 23 illustrates pharmacodynamic data utilizing at least one of the above-described drug delivery devices. As shown, the data indicates a drop in blood glucose after dosing 4 international units of insulin. Thus, Fig. 23 illustrates changes in blood glucose levels upon hooking and delivering an active drug substance, e.g. insulin.

The use of the terms "first", "second", "third" and "fourth", "primary", "secondary", "tertiary" etc. does not imply any particular order, but are included to identify individual elements. Moreover, the use of the terms "first", "second", "third" and "fourth", "primary", "secondary", "tertiary" etc. does not denote any order or importance, but rather the terms "first", "second", "third" and "fourth", "primary", "secondary", "tertiary" etc. are used to distinguish one element from another. Note that the words "first", "second", "third" and "fourth", "primary", "secondary", "tertiary" etc. are used here and elsewhere for labelling purposes only and are not intended to denote any specific spatial or temporal ordering. Furthermore, the labelling of a first element does not imply the presence of a second element and vice versa.

It is to be noted that the word "comprising" does not necessarily exclude the presence of other elements or steps than those listed.

It is to be noted that the words "a" or "an" preceding an element do not exclude the presence of a plurality of such elements.

It should further be noted that any reference signs do not limit the scope of the claims, that the exemplary embodiments may be implemented at least in part by means of both hardware and software, and that several "means", "units" or "devices" may be represented by the same item of hardware.

Although features have been shown and described, it will be understood that they are not intended to limit the claimed invention, and it will be made obvious to those skilled in the art that various changes and modifications may be made without departing from the scope of the claimed invention. The specification and drawings are, accordingly to be regarded in an illustrative rather than restrictive sense.

### LIST OF REFERENCES

- 2, 2A, 2B, 2C, 2D: drug delivery device
- 4: first body part
- 4A: first primary body part
- 4B: first secondary body part
- 6: first end of first body part
- 8: second end of first body part
- 10: second body part
- 10A: second primary body part
- 10B: second secondary body part
- 12: first end of second body part
- 14: second end of second body part
- 16: actuator mechanism
- 16A: resilient part
- 18: first part of resilient part
- 20: second part of resilient part
- 22: outer periphery of spiral torsion spring
- 24: center part of spiral torsion spring
- 26: inner volume
- 28: inner surface
- 30: first engagement part
- 30': first engagement part
- 32: second engagement part
- 34: slit
- 36: first attachment part
- 36A: first base
- 37: first needle
- 38: first proximal end of first attachment part
- 40: first distal end of first attachment part
- 42: outer surface of first body part
- 44: second attachment part
- 45: second needle
- 46: second proximal end of second attachment part
- 48: second distal end of second attachment part
- 50: outer surface of second body part
- 52: sharp tip
- 56: opening
- 64: first primary recess in first body part
- 66: second primary recess in second body part
- 68: first compartment
- 69: second compartment
- 70: locking mechanism
- 72: first locking element
- 74: bodily tissue
- 76: housing
- 78: frame part
- 80: first opening in first body part
- 82: second opening in second body part
- 100: pharmaceutical composition
- 102: first locking band
- 103: first cover band
- 104: first attachment part
- 106: second attachment part
- 108: first body recess
- 112: locking protrusion
- 114: mating feature
- 116: joint
- A: central axis/primary axis
- B: rotational direction
- C: rotational direction
- D: plane axis
- X_1: first attachment axis
- X_R_1: first rotation axis
- X_2: second attachment axis
- X_R_2: second rotation axis
- α: angle

## Claims

1. A drug delivery device (2, 2A, 2B, 2C, 2D) for oral administration, the drug delivery device having a central axis and comprising:
a first body part (4);
a first attachment part (36) attached to the first body part,
**characterized in that** the first attachment part (36) has a first distal end (40) provided with a tip configured to penetrate a biological tissue; and
the drug delivery device comprising:
a second attachment part (44) having a second distal end (48);
a second body part (10), wherein the second attachment part (44) is attached to the second body part (10); and
an actuator mechanism (16) configured to move the first distal end (40) towards the second distal end (48), and wherein the actuator mechanism (16) is configured to rotate the first body part (4) in relation to the second body part (10) about a primary axis of the drug delivery device.

2. Drug delivery device according to claim 1, wherein the actuator mechanism (16) comprises a resilient part (16A) configured to apply force to the first body part (4) and/or the second body part (10).

3. Drug delivery device according to claim 2, wherein a first part 18 of the resilient part (16A) is connected to the first body part (4) and a second part (20) of the resilient part (16A) is connected to the second body part (10).

4. Drug delivery device according to any of claims 1-3, wherein the first attachment part (36) extends in a direction away from the first body part (4).

5. Drug delivery device according to any of claims 1-4, wherein the second attachment (44) part extends in a direction away from the second body part (10).

6. Drug delivery device according to any of claims 1-5, wherein the first attachment part (36) has a first attachment axis (X_1), and wherein a distance between the first attachment axis (X_1) and the primary axis is larger than 0.5 mm.

7. Drug delivery device according to any of claims 1-6, wherein the second attachment part (44) has a second attachment axis (X_2), and wherein a distance between the second attachment axis and the primary axis is larger than 0.5 mm.

8. Drug delivery according to any of claims 1-7, wherein the first body part (4) is configured to rotate in a first direction and the second body part (10) is configured to rotate in a second direction opposite to the first direction.

9. Drug delivery device according to any of claims 1-8, wherein the second distal end of the second attachment part is provided with a tip (52) configured to penetrate a biological tissue.

10. Drug delivery device according to any of claims 1-8, wherein the second distal end of the second attachment part is provided with a gripping part configured to grip a biological tissue.

11. Drug delivery device according to any of claims 1-10, wherein the drug delivery device comprises a first compartment (68), the drug delivery device being configured to deliver an active drug substance from the first compartment (68) to the surroundings of the drug delivery device.

12. Drug delivery device according to claim 11, wherein the first compartment (68) is arranged in the first attachment part (36).

13. Drug delivery device according to any of claims 1-12, wherein the actuator mechanism (16) is configured to move the first distal end from a first primary angular position of first primary position to a first secondary angular position of first secondary position in relation to a first proximal end of the first attachment part, wherein an angle between the first primary angular position and the first secondary angular position is larger than 10 degrees.

14. Drug delivery device according to any of claim 1-13, wherein the first attachment part (36) is rotationally attached to the first body part (4) and configured to rotate about a first rotation axis perpendicular or parallel to the primary axis.

15. Pharmaceutical composition comprising a drug delivery device according to any of claims 1-14 and an active drug substance.

## Patentansprüche

1. Arzneimittelabgabevorrichtung (2, 2A, 2B, 2C, 2D) zur oralen Verabreichung, wobei die Arzneimittelabgabevorrichtung eine zentrale Achse aufweist und Folgendes umfasst:
ein erstes Körperteil (4);
ein an dem ersten Körperteil befestigtes erstes Befestigungsteil (36),
**dadurch gekennzeichnet, dass** das erste Befestigungsteil (36) ein erstes distales Ende (40) aufweist, das mit einer zum Eindringen in ein biologisches Gewebe ausgelegten Spitze versehen ist; und
wobei die Arzneimittelabgabevorrichtung Folgendes umfasst:
ein zweites Befestigungsteil (44) mit einem zweiten distalen Ende (48);
ein zweites Körperteil (10), wobei das zweite Befestigungsteil (44) an dem zweiten Körperteil (10) befestigt ist; und
einen Aktuatormechanismus (16), der zum Bewegen des ersten distalen Endes (40) in Richtung des zweiten distalen Endes (48) ausgelegt ist, und wobei der Aktuatormechanismus (16) zum Drehen des ersten Körperteils (4) in Bezug auf das zweite Körperteil (10) um eine primäre Achse der Arzneimittelabgabevorrichtung ausgelegt ist.

2. Arzneimittelabgabevorrichtung nach Anspruch 1, wobei der Aktuatormechanismus (16) ein elastisches Teil (16A) umfasst, das zum Ausüben von Kraft auf das erste Körperteil (4) und/oder das zweite Körperteil (10) ausgelegt ist.

3. Arzneimittelabgabevorrichtung nach Anspruch 2, wobei ein erstes Teil 18 des elastischen Teils (16A) mit dem ersten Körperteil (4) verbunden ist und ein zweites Teil (20) des elastischen Teils (16A) mit dem zweiten Körperteil (10) verbunden ist.

4. Arzneimittelabgabevorrichtung nach einem der Ansprüche 1-3, wobei sich das erste Befestigungsteil (36) in eine Richtung weg von dem ersten Körperteil (4) erstreckt.

5. Arzneimittelabgabevorrichtung nach einem der Ansprüche 1-4, wobei sich das zweite Befestigungsteil (44) in eine Richtung weg von dem zweiten Körperteil (10) erstreckt.

6. Arzneimittelabgabevorrichtung nach einem der Ansprüche 1-5, wobei das erste Befestigungsteil (36) eine erste Befestigungsachse (X_1) aufweist und wobei ein Abstand zwischen der ersten Befestigungsachse (X_1) und der primären Achse größer ist als 0,5 mm.

7. Arzneimittelabgabevorrichtung nach einem der Ansprüche 1-6, wobei das zweite Befestigungsteil (44) eine zweite Befestigungsachse (X_2) aufweist und wobei ein Abstand zwischen der zweiten Befestigungsachse und der primären Achse größer ist als 0,5 mm.

8. Arzneimittelabgabe nach einem der Ansprüche 1-7, wobei das erste Körperteil (4) zum Drehen in eine erste Richtung ausgelegt ist und das zweite Körperteil (10) zum Drehen in eine zweite der ersten Richtung entgegengesetzte Richtung ausgelegt ist.

9. Arzneimittelabgabevorrichtung nach einem der Ansprüche 1-8, wobei das zweite distale Ende des zweiten Befestigungsteils mit einer zum Durchdringen eines biologischen Gewebes ausgelegten Spitze (52) versehen ist.

10. Arzneimittelabgabevorrichtung nach einem der Ansprüche 1-8, wobei das zweite distale Ende des zweiten Befestigungsteils mit einem Greifen eines biologischen Gewebes ausgelegten Griffteil versehen ist.

11. Arzneimittelabgabevorrichtung nach einem der Ansprüche 1-10, wobei die Arzneimittelabgabevorrichtung eine erste Kammer (68) umfasst, wobei die Arzneimittelabgabevorrichtung zum Abgeben eines Wirkstoffs aus der ersten Kammer (68) in die Umgebung der Arzneimittelabgabevorrichtung ausgelegt ist.

12. Arzneimittelabgabevorrichtung nach Anspruch 11, wobei die erste Kammer (68) in dem ersten Befestigungsteil (36) angeordnet ist.

13. Arzneimittelabgabevorrichtung nach einem der Ansprüche 1-12, wobei der Aktuatormechanismus (16) zum Bewegen des ersten distalen Endes von einer ersten primären Winkelposition einer ersten primären Position zu einer ersten sekundären Winkelposition einer ersten sekundären Position in Bezug auf ein erstes proximales Ende des ersten Befestigungsteils ausgelegt, wobei ein Winkel zwischen der ersten primären Winkelposition und der ersten sekundären Winkelposition größer ist als 10 Grad.

14. Arzneimittelabgabevorrichtung nach einem der Ansprüche 1-13, wobei das erste Befestigungsteil (36) drehbar an dem ersten Körperteil (4) befestigt ist und zum Drehen um eine erste Drehachse senkrecht oder parallel zu der primären Achse ausgelegt ist.

15. Pharmazeutische Zusammensetzung, umfassend eine Arzneimittelabgabevorrichtung nach einem der Ansprüche 1-14 und einen Wirkstoff.

## Revendications

1. Dispositif d'administration de médicament (2, 2A, 2B, 2C, 2D) pour administration orale, le dispositif d'administration de médicament ayant un axe central et comprenant :
une première partie de corps (4) ;
une première partie de fixation (36) fixée à la première partie de corps,
**caractérisée en ce que** la première partie de fixation (36) a une première extrémité distale (40) pourvue d'une pointe configurée pour pénétrer un tissu biologique ; et
le dispositif d'administration de médicament comprenant :
une seconde partie de fixation (44) ayant une seconde extrémité distale (48) ;
une seconde partie de corps (10), dans lequel la seconde partie de fixation (44) est fixée à la seconde partie de corps (10) ; et
un mécanisme d'actionnement (16) configuré pour déplacer la première extrémité distale (40) vers la seconde extrémité distale (48), et dans lequel le mécanisme d'actionnement (16) est configuré pour faire tourner la première partie de corps (4) par rapport à la seconde partie de corps (10) autour d'un axe principal du dispositif d'administration de médicament.

2. Dispositif d'administration de médicament selon la revendication 1, dans lequel le mécanisme d'actionnement (16) comprend une partie élastique (16A) configurée pour appliquer une force à la première partie de corps (4) et/ou à la seconde partie de corps (10).

3. Dispositif d'administration de médicament selon la revendication 2, dans lequel une première partie 18 de la partie élastique (16A) est connectée à la première partie de corps (4) et une seconde partie (20) de la partie élastique (16A) est connectée à la seconde partie de corps (10).

4. Dispositif d'administration de médicament selon l'une quelconque des revendications 1 à 3, dans lequel la première partie de fixation (36) se prolonge dans une direction s'éloignant de la première partie de corps (4).

5. Dispositif d'administration de médicament selon l'une quelconque des revendications 1 à 4, dans lequel la seconde partie de fixation (44) se prolonge dans une direction s'éloignant de la seconde partie de corps (10).

6. Dispositif d'administration de médicament selon l'une quelconque des revendications 1 à 5, dans lequel la première partie de fixation (36) a un premier axe de fixation (X_1), et dans lequel une distance entre le premier axe de fixation (X_1) et l'axe principal est supérieure à 0,5 mm.

7. Dispositif d'administration de médicament selon l'une quelconque des revendications 1 à 6, dans lequel la seconde partie de fixation (44) a un second axe de fixation (X_2), et dans lequel une distance entre le second axe de fixation et l'axe principal est supérieure à 0,5 mm.

8. Dispositif d'administration de médicament selon l'une quelconque des revendications 1 à 7, dans lequel la première partie de corps (4) est configurée pour tourner dans une première direction et la seconde partie de corps (10) est configurée pour tourner dans une seconde direction opposée à la première direction.

9. Dispositif d'administration de médicament selon l'une quelconque des revendications 1 à 8, dans lequel la seconde extrémité distale de la seconde partie de fixation est pourvue d'une pointe (52) configurée pour pénétrer un tissu biologique.

10. Dispositif d'administration de médicament selon l'une quelconque des revendications 1 à 8, dans lequel la seconde extrémité distale de la seconde partie de fixation est pourvue d'une partie de préhension configurée pour saisir un tissu biologique.

11. Dispositif d'administration de médicament selon l'une quelconque des revendications 1 à 10, dans lequel le dispositif d'administration de médicament comprend un premier compartiment (68), le dispositif d'administration de médicament étant configuré pour administrer une substance médicamenteuse active depuis le premier compartiment (68) vers l'environnement du dispositif d'administration de médicament.

12. Dispositif d'administration de médicament selon la revendication 11, dans lequel le premier compartiment (68) est disposé dans la première partie de fixation (36).

13. Dispositif d'administration de médicament selon l'une quelconque des revendications 1 à 12, dans lequel le mécanisme d'actionnement (16) est configuré pour déplacer la première extrémité distale d'une première position angulaire primaire de première position primaire à une première position angulaire secondaire de première position secondaire par rapport à une première extrémité proximale de la première partie de fixation, dans lequel un angle entre la première position angulaire primaire et la première position angulaire secondaire est supérieur à 10 degrés.

14. Dispositif d'administration de médicament selon l'une quelconque des revendications 1 à 13, dans lequel la première partie de fixation (36) est fixée de manière rotative à la première partie de corps (4) et est configurée pour tourner autour d'un premier axe de rotation perpendiculaire ou parallèle à l'axe principal.

15. Composition pharmaceutique comprenant un dispositif d'administration de médicament selon l'une quelconque des revendications 1 à 14 et une substance médicamenteuse active.
